# EUROPEAN PATENT APPLICATION

(11) **EP 4 029 496 A1**
(43) Date of publication of application: **20.07.2022**
(21) Application number: 21382036.8
(22) Date of filing: 18.01.2021
(51) Int. Cl.: A61K 31/05, A61K 31/365, A61P 39/00, A61P 39/06

(54) **PTEROSTILBENE AND SILIBININ FOR PREVENTING, AMELIORATING OR REDUCING RADIATION-INDUCED DISEASES**

(71) Applicant: Universitat de València, 46010 Valencia (ES); Fundación Para la Investigación del Hospital Universitario y Politécnico La Fe de la Comunidad Valenciana, 46026 Valencia (ES)
(72) Inventor: Estrela Arigüel, José María, 46010 Valencia (ES); Obrador Pla, María Elena, 46010 Valencia (ES); Salvador Palmer, María Rosario, 46010 Valencia (ES); Montoro Pastor, Alegría, 46026 VALENCIA (ES)
(74) Representative: Hoffmann Eitle

(57) **Abstract**

The present invention relates to compositions comprising Pterostilbene, or a pharmaceutically acceptable salt thereof, in combination with Silibinin, or a pharmaceutically acceptable salt thereof, and their use in the treatment, prevention, amelioration or reduction of diseases induced by ionizing radiation in a subject. The compositions and the uses can further comprise at least one or more radiomitigator compound/s, preferably NAD+ boosters and/or FSL-1.

## Description

### TECHNICAL FIELD OF THE INVENTION

The present invention relates to the field of medicine. In particular, the present invention relates to the field of compositions and methods for preventing, ameliorating or reducing radiation-induced diseases.

### BACKGROUND OF THE INVENTION

Nowadays, ionizing radiations are used for various medical aims. X and γ rays damage cells mainly by direct ionization of DNA and other cellular targets and by indirect effects through reactive oxygen species (ROS). The difference between γ rays and X-rays is how they are produced. Gamma rays originate from the settling process of an excited nucleus of a radionuclide after it undergoes radioactive decay whereas X-rays are produced when electrons strike a target or when electrons rearrange within an atom. X and γ rays are both types of high energy (high frequency) electromagnetic radiation. They are packets of energy (photons) that have no charge or mass. Both, X and γ rays have the same properties and health effects.

Electromagnetic radiation, including X-rays and γ-rays, damages the cells directly by ionization of DNA and other molecules, and also indirectly by the generation of reactive oxygen species. The response to radiation exposure depends on the cell type and dose of radiation, inherent tissue sensitivity and repair, and modulating intracellular factors that include cell cycle status, O₂ pressure, and levels of thiols and other antioxidants. However, the radiation protection and mitigation strategies are scarce and inefficient. Annually, millions of cancer patients undergo radiotherapy during their course of treatment and some radiological or nuclear events in recent years pose a threat to people, hence the need for radiation protection or mitigation strategies.

The development of protective agents against exposure to harmful radiations has been a subject of intense investigation for decades. Effective and safe radioprotectants are not available. To date, no drug has been developed for the treatment of high levels exposition of ionizing radiation, such as the one that causes acute hematopoietic and gastrointestinal radiation syndromes (ARS).

Radioprotectors are compounds that can reduce the direct damage caused by radiation. Radiomitigators are compounds that are able to minimize toxicity even after radiation has been delivered. In general, ideal radioprotectors or radiomitigators should be stable, with the possibility of an easy administration, with no relevant toxicity, and should protect normal tissues that are considered sensitive such that acute or late toxicities in these tissues are either dose-limiting or responsible for a significant reduction in quality of life (causing e.g. mucositis, pneumonitis, myelopathy, xerostomia, proctitis, fibrosis, or leukoencephalopathy).

A wide range of phytochemicals (compounds produced by plants, generally to help them thrive or thwart competitors, predators, or pathogens) are antioxidants and, thus, potentially radioprotective. However, their radioprotective or radiomitigator properties are usually effective during a short period of time, usually limited to a maximum of one month after radiation occurred. Therefore, new compounds and compositions that are able to treat, reduce, prevent or ameliorate the harmful effect of radiation in a subject during a continued period of times are desirable.

The present invention provides compositions and uses thereof for the long-term protection and mitigation of diseases caused by radiation exposure.

### DESCRIPTION OF THE FIGURES

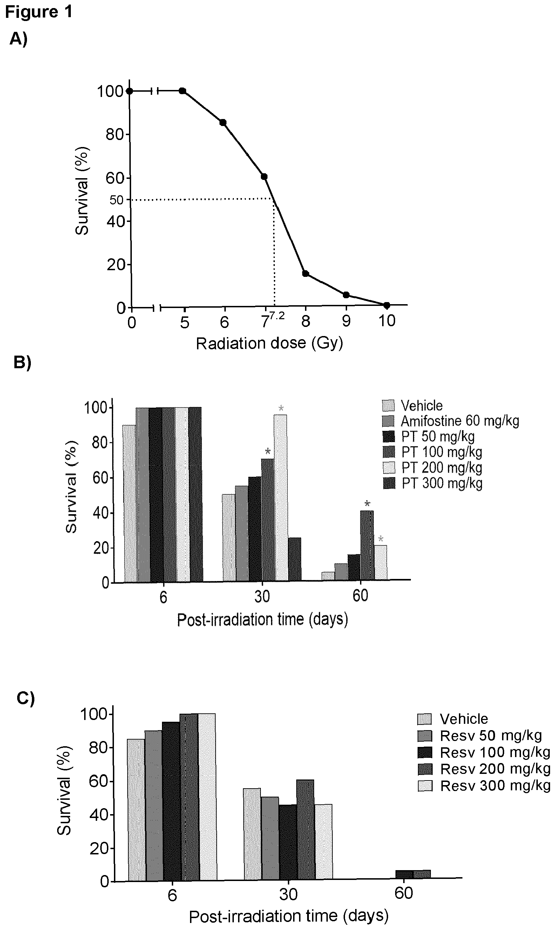

**Figure 1****. A)** Radiation dose-response for a 30-day survival test in Swiss albino mice subjected to whole-body γ irradiation **B)** Sixty-days survival of mice treated with γ rays (LD50/30) and pterostilbene (PT) at different doses. Amifostine was used as a control approved radioprotector C) Sixty-days survival of mice treated with γ rays (LD50/30) and resveratrol (Resv) at different doses. Compounds were administered orally. In all cases, twenty mice were used per group.

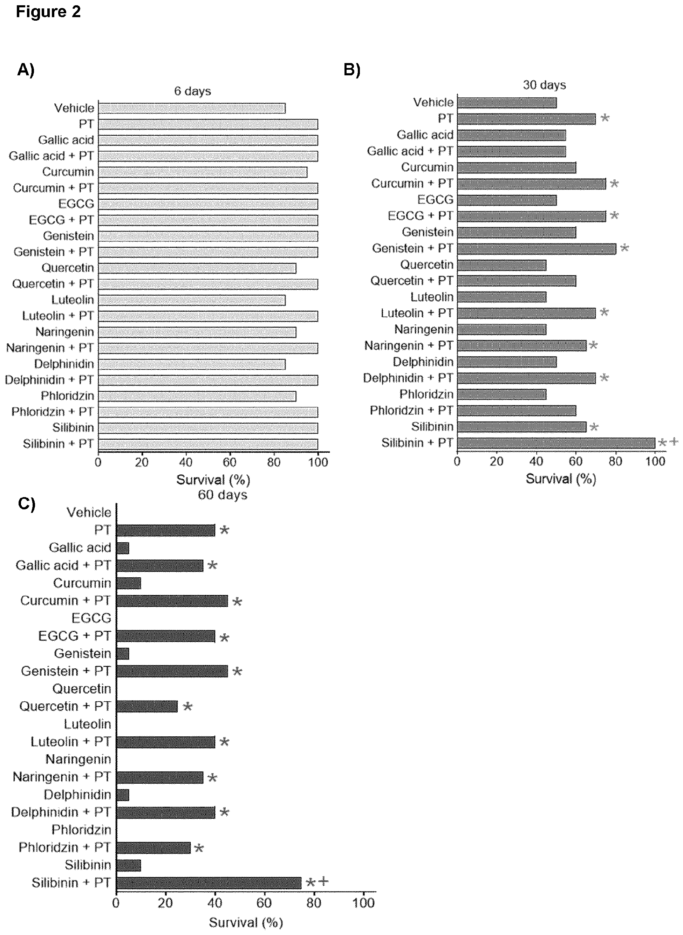

**Figure 2****.** Combination of pterostilbene (PT) and other polyphenols to prevent the lethality induced by γ radiation (LD50/30) at **A)** 6 days, **B)** 30 days and C) 60 days post-irradiation. Doses for each phytochemical were selected from the max non-toxic doses published. Compounds were administered orally. In all cases, twenty mice were used per group.

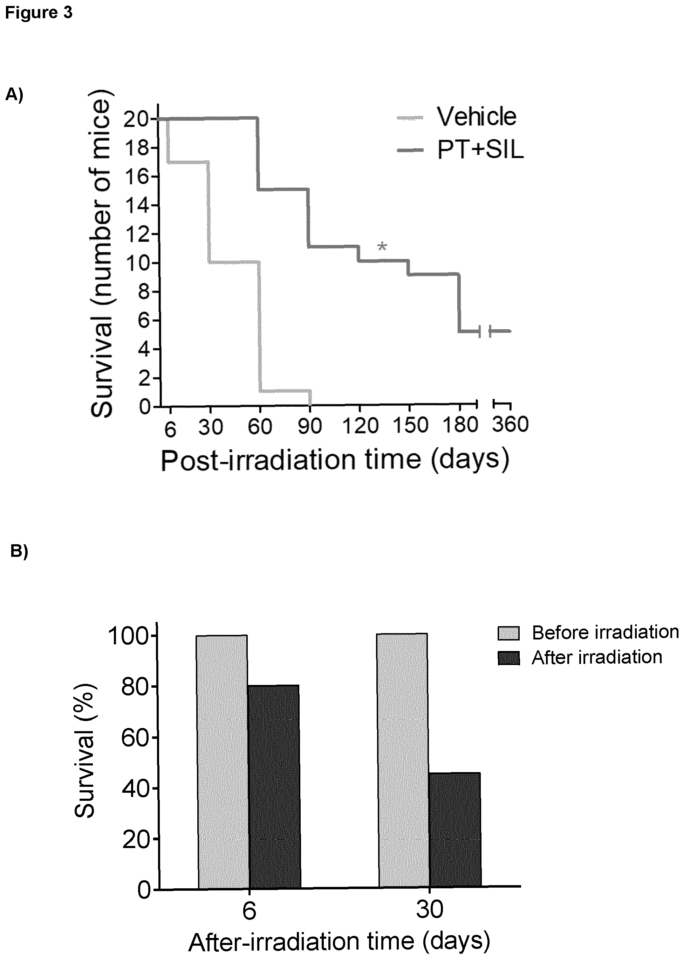

**Figure 3****. A)** Effect of the combination of pterostilbene (PT) and silibinin (SIL) on the long-term survival of mice subjected to whole-body γ irradiation (LD50/30). **B)** Radioprotection elicited by the combination of PT and SIL: Comparison between administration of the polyphenols before or after the γ irradiation. Compounds were administered IP. In all cases, twenty mice were used per group.

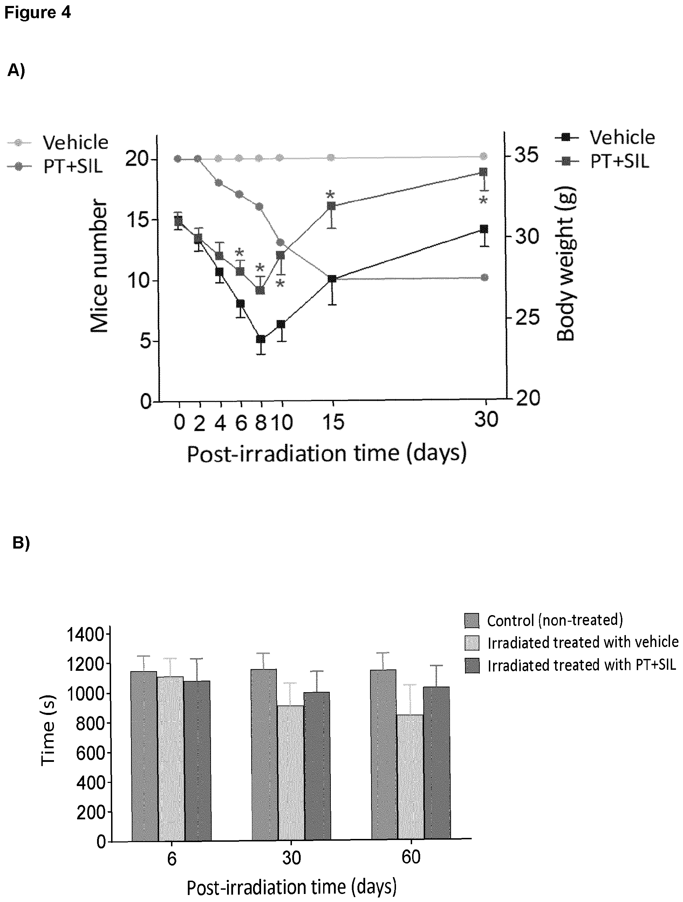

**Figure 4****. A)** Effect of pterostilbene (PT) and silibinin (SIL) on the survival of γ-irradiated (LD50/30) mice and the effect on body weight. **B)** Effect of PT and SIL administration on the whole-body γ radiation-induced alteration of neuromotor functions in mice. Compounds were administered via IP. In all cases, twenty mice were used per group.

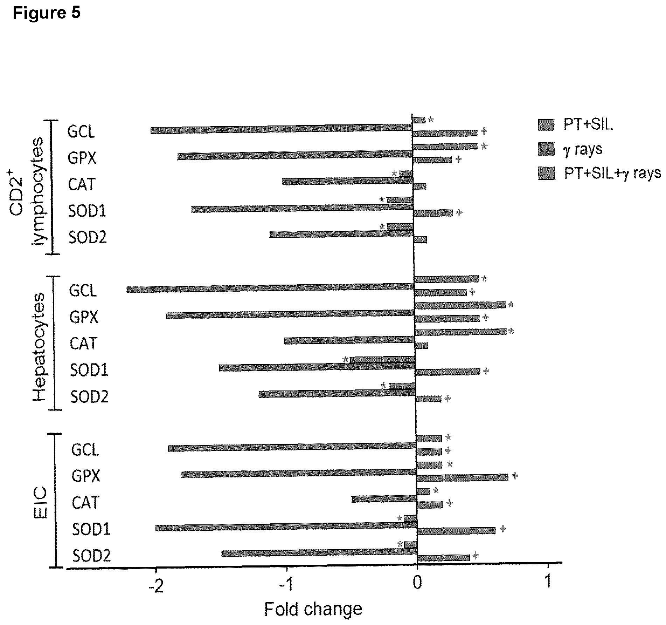

**Figure 5****.** Protective effect of pterostilbene (PT) and silibinin (SIL) on the γ radiation-induced oxidative damage expresion (RT-PCR) of different antioxidant defense-related enzyme activities in isolated cells. GCL, g-glutamyl-cysteine ligase; GPX, glutathione peroxidase; CAT, catalase; SOD, superoxide dismutase; EIC, epitelial intestinal cells. Fold change versus the expression rate of control (untreated) cells (n=5 per molecule, cell type, and treatment).

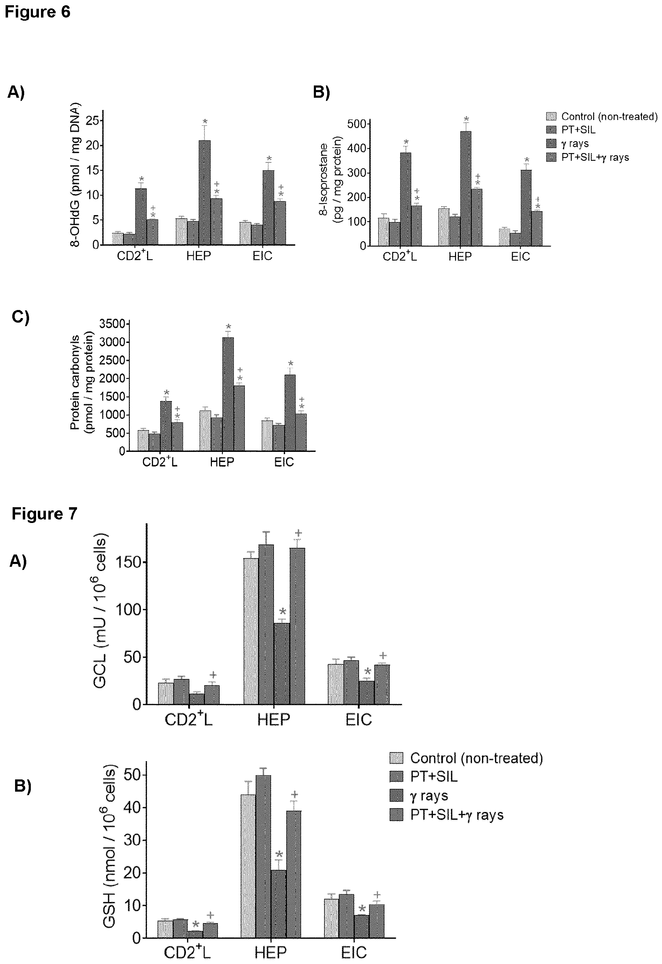

**Figure 6****.** Protective effect of pterostilbene (PT) and silibinin (SIL) on the γ radiation-induced oxidative damage in different molecular markers. **A)** 8-OHdG, **B)** 8-isoprostane and **C)** protein carbonyls (n=5 per molecule, cell type, and treatment).
**Figure 7****.** Protective effect of pterostilbene (PT) and silibinin (SIL) on the γ radiation-induced oxidative damage in the glutathione-related antioxidant defense in **A)** γ-glutamyl- cysteine ligase, **B)** glutathione (n=5 per molecule, cell type, and treatment).

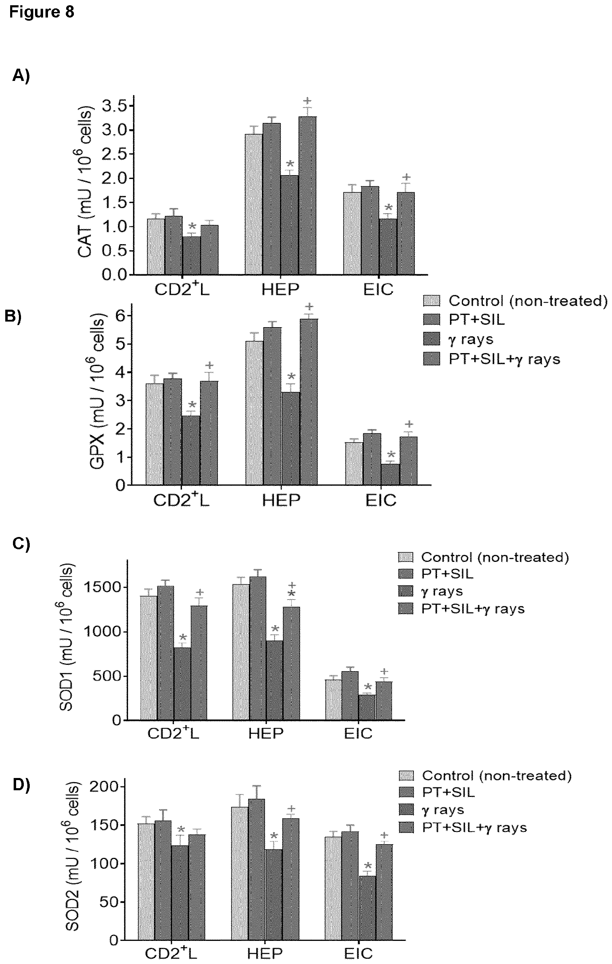

**Figure 8****.** Protective effect of pterostilbene (PT) and silibinin (SIL) on the γ radiation-induced oxidative damage in different antioxidant defense-related enzyme activities: **A)** catalase, **B)** glutathione peroxidase and **C)** and **D)** superoxide dismutases 1 and 2 (n=5 per molecule, cell type, and treatment).

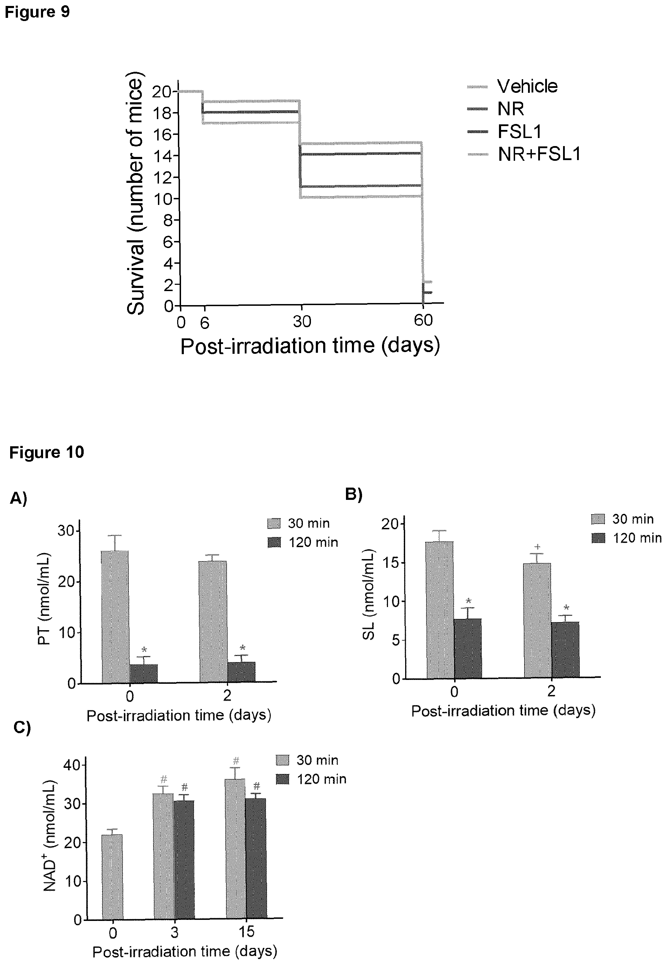

**Figure 9****.** Effect of nicotinamide riboside (NR) and fibroblast-stimulating lipopeptide (FSL-1) on the γ irradiation (LD50/30)-induced mortality. NR was administered daily for 30 days, one single dose (oral) per day, and starting right after (60 min) the irradiation. FSL-1 was administered IP, only once, 24 h after the irradiation. In all cases, twenty mice were used per group.
**Figure 10****. A)** Pterostilbene (PT), **B)** Silibinin (SIL) and **C)** NAD+ levels in the circulating blood of treated and irradiated mice (6 mice per group in all cases).

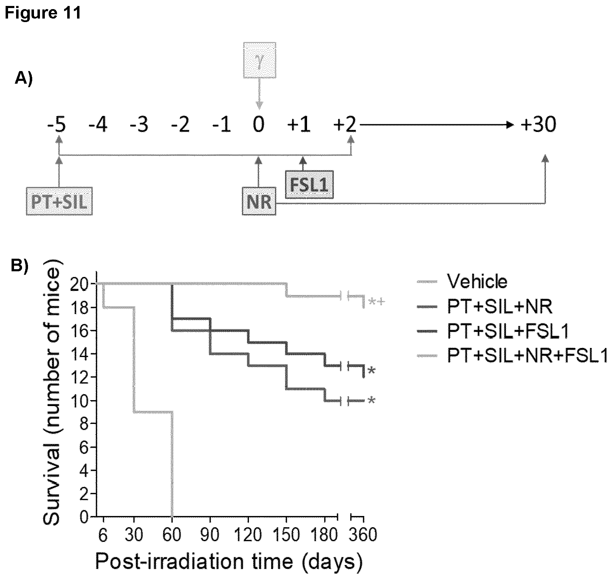

**Figure 11****.** Addition of potential radiomitigators (nicotinamide riboside, NR; and fibroblast-stimulating lipopeptide-1, FSL-1) to the radioprotective combination of pterostilbene (PT) and silibinin (SIL). **A)** administration scheme, **B)** survival versus post-irradiation time in days. In all cases, twenty mice were used per group.

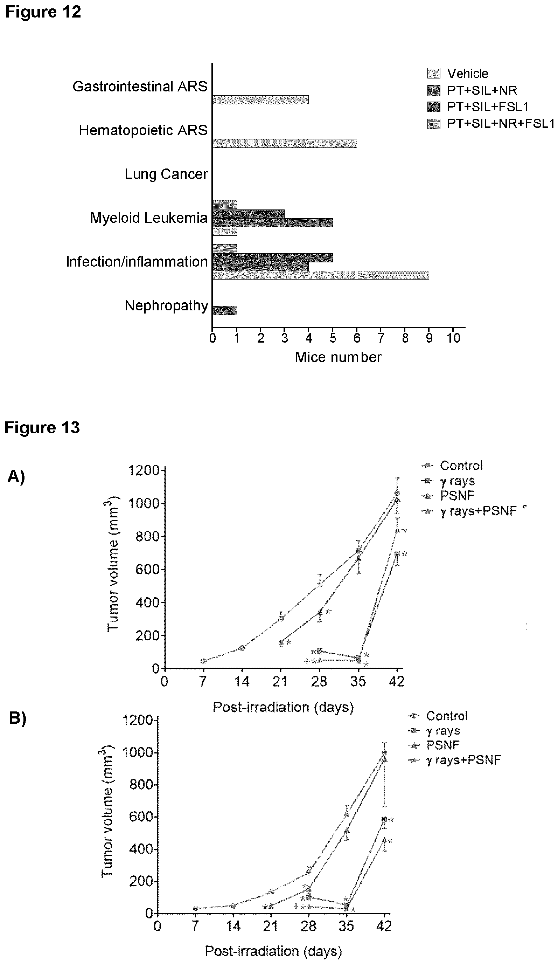

**Figure 12****.** Causes of death in γ irradiated mice (LD50/30) and the effect of the combined administration of radioprotectors and radiomitigators (PT, SIL, NR and FSL-1). In all cases, twenty mice were studied per group.
Figure 13. Combined administration of the radioprotecting/radiomitigating combination PT + SIL + NR + FSL-1 (PSNF) and X rays to **A)** growing human A549 (lung adenocarcinoma) and **B)** MDA-MB-231 (triple negative mammary carcinoma) xenografts. In all cases, five mice were used per group.

### BRIEF DESCRIPTION OF THE INVENTION

In a first aspect, the present invention relates to Pterostilbene, or a pharmaceutically acceptable salt thereof, for use in the prevention, amelioration or reduction of diseases induced by ionizing radiation in a subject, in combination with Silibinin, or a pharmaceutically acceptable salt thereof, wherein the administration is carried out before the radiation, or after the radiation, or during the radiation; and wherein the combination is herein understood as the simultaneous or sequential administration, in any order, of a) Pterostilbene, or a pharmaceutically acceptable salt thereof, and b) Silibinin, or a pharmaceutically acceptable salt thereof, to said subject.

The Pterostilbene for use according to the first aspect, wherein Pterostilbene and Silibinin, or pharmaceutically acceptable salts thereof, are administered in a sole pharmaceutical composition or in separate pharmaceutical compositions.

The Pterostilbene for use according to the first aspect, wherein the administration of Pterostilbene and Silibinin, or pharmaceutically acceptable salts thereof, is topical, oral or parenteral.

The Pterostilbene for use according to the first aspect, wherein Pterostilbene and Silibinin, or pharmaceutically acceptable salts thereof, are administrated several times simultaneously or sequentially before and/or after radiation, or both.

The Pterostilbene for use according to the first aspect, wherein the combination is in a dosage capable of providing a radioprotective effect.

The Pterostilbene for use according to the first aspect, wherein Pterostilbene, or a pharmaceutically acceptable salt thereof, is administered at a dosage from 300 mg/kg to 30 mg/kg, preferably 100 mg/kg, and Silibinin, or a pharmaceutically acceptable salt thereof, is administered at a dosage from 200 mg/kg to 35 mg/kg of Silibinin, preferably 70 mg/kg.

The Pterostilbene for use according to the first aspect, wherein the use further comprises the administration in combination with at least one or more radiomitigator compound/s, wherein the combination is herein understood as the simultaneous or sequential administration of a) Pterostilbene, or a pharmaceutically acceptable salt thereof, b) Silibinin, or a pharmaceutically acceptable salt thereof, and c) at least one or more radiomitigator compound/s, or any pharmaceutically acceptable salt thereof, to said subject.

The Pterostilbene for use according to the first aspect, wherein the at least one or more radiomitigator compound/s is a NAD+ booster and/or a Fibroblast-Stimulating Lipopeptide, or any pharmaceutically acceptable salts thereof.

The Pterostilbene for use according to the first aspect, wherein the use further comprises the administration in combination with at least two or more radiomitigator compounds, wherein the at least two or more radiomitigator compounds are NAD+ booster and a Fibroblast-Stimulating Lipopeptide, or any pharmaceutically acceptable salts thereof.

The Pterostilbene for use according to the first aspect, wherein the NAD+ booster, or any pharmaceutically acceptable salt thereof, is administered at a dosage from 400 mg/kg to 100 mg/kg, preferably 185 mg/kg, and/or wherein the Fibroblast-Stimulating Lipopeptide, or a pharmaceutically acceptable salt thereof, is administered at a dosage from 1mg/kg to 0,1 mg/kg, preferably 0.25 mg/kg.

The Pterostilbene for use according to the first aspect, wherein the NAD+ booster is Nicotinamide Riboside.

The Pterostilbene for use according to the first aspect, wherein the diseases induced by ionizing radiation are related to DNA damage, radiation-induced cytotoxicity, genotoxicity and/or oxidative cellular damage.

The Pterostilbene for use according to the first aspect, wherein the diseases induced by ionizing radiation are acute radiation syndrome and/or chronic radiation syndrome.

The Pterostilbene for use according to the first aspect, wherein the ionizing radiation is gamma rays or X-rays.

The Pterostilbene for use according to the first aspect, wherein the Pterostilbene is Pterostilbene phosphate disodium salt and/or wherein the Silibinin is silibinin-C-2',3-dihydrogen succinate, disodium salt.

In a second aspect, the present invention relates to a pharmaceutical composition comprising a) Pterostilbene, or a pharmaceutically acceptable salt thereof, and b) Silibinin, or a pharmaceutically acceptable salt thereof.

The pharmaceutical composition according to the second aspect, wherein the dosage of Pterostilbene, or a pharmaceutically acceptable salt thereof, present in said composition is from 300 mg/kg to 30 mg/kg, preferably 100 mg/kg, and wherein the dosage of Silibinin, or a pharmaceutically acceptable salt thereof, present in said composition is from 200mg/kg to 35 mg/kg of Silibinin, preferably 70 mg/kg.

The pharmaceutical composition according to the second aspect, wherein the pharmaceutical composition further comprises at least one or more radiomitigator compound/s, preferably selected from
a) a NAD+ booster, and/or
b) a Fibroblast-Stimulating Lipopeptide-1, or any pharmaceutically acceptable salts thereof.

The pharmaceutical composition according to the second aspect, wherein the pharmaceutical composition further comprises at least two or more radiomitigators compounds, wherein the at least two or more radiomitigator compounds are a NAD+ booster and a Fibroblast-Stimulating Lipopeptide-1, or any pharmaceutically acceptable salts thereof.

The pharmaceutical composition according to the second aspect, wherein the NAD+ booster, or a pharmaceutically acceptable salt thereof, is present in said composition at a dosage from 400 mg/kg to 100 mg/kg, preferably 185 mg/kg, and/or wherein the Fibroblast-Stimulating Lipopeptide-1, or a pharmaceutically acceptable salt thereof, is present in said composition at a dosage from 1mg/kg to 0,1 mg/kg, preferably 0.25 mg/kg.

The pharmaceutical composition according to the second aspect, wherein the NAD+ booster is Nicotinamide Riboside.

The pharmaceutical composition according to the second aspect, wherein said composition further comprises a pharmaceutically acceptable carrier, additive and/or excipient.

The pharmaceutical composition according to the second aspect, wherein the Pterostilbene is Pterostilbene phosphate disodium salt and/or wherein the Silibinin is silibinin-C-2',3-dihydrogen succinate, disodium salt.

In a third aspect, the present invention relates to a kit of parts comprising at least two recipients comprising at least Pterostilbene and Silibinin, or any pharmaceutically acceptable salts thereof, and optionally at least one or more radiomitigator compound/s.

The kit of parts according to the third aspect, wherein the Pterostilbene, or a pharmaceutically acceptable salt thereof, is present at a dosage from 300 mg/kg to 30 mg/kg, preferably 100 mg/kg, and Silibinin, or a pharmaceutically acceptable salt thereof, is present at a dosage from 200mg/kg to 35 mg/kg of Silibinin, preferably 70 mg/kg.

The kit of parts according to the third aspect, wherein the optionally at least one or more radiomitigator compound/s is selected from the group of:
a) NAD+ boosters, and/or
b) a Fibroblast-Stimulating Lipopeptide-1, or any pharmaceutically acceptable salts thereof.

The kit of parts according to the third aspect, wherein the NAD+ boosters, or a pharmaceutically acceptable salt thereof, is present at a dosage from 400 mg/kg to 100 mg/kg, preferably 185 mg/kg, and/or wherein the Fibroblast-Stimulating Lipopeptide, or a pharmaceutically acceptable salt thereof, is present at a dosage from 1mg/kg to 0,1 mg/kg, preferably 0.25 mg/kg.

The kit of parts according to the third aspect, wherein the NAD+ booster is Nicotinamide Riboside.

The kit of parts according to the third aspect, wherein the Pterostilbene is Pterostilbene phosphate disodium salt and/or wherein the Silibinin is silibinin-C-2',3-dihydrogen succinate, disodium salt.

In a fourth aspect, the present invention relates to a method for preventing, ameliorating or reducing diseases induced by ionizing radiation in a subject, comprising administering Pterostilbene in combination with Silibinin, or pharmaceutically acceptable salts thereof, wherein said administration is performed before the radiation, or after the radiation, or during the radiation; and wherein the combination is herein understood as the simultaneous or sequential administration of a) Pterostilbene, or a pharmaceutically acceptable salt thereof, and b) Silibinin, or a pharmaceutically acceptable salt thereof, to said subject.

The method according to the fourth aspect, further comprising the administration of at least one radiomitigator compound, wherein the at least one or more radiomitigator compound are preferably selected from
a) NAD+ boosters, and/or
b) a Fibroblast-Stimulating Lipopeptide, or any pharmaceutically acceptable salts thereof.

The method according to the fourth aspect, wherein the NAD+ booster is Nicotinamide Riboside.

### DESCRIPTION OF THE INVENTION

### GENERAL DEFINITIONS

It must be noted that, as used herein, the singular forms "a", "an", and "the", include plural references unless the context clearly indicates otherwise. Further, unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

The term "about" when referred to a given amount or quantity is meant to include deviations of plus or minus five percent.

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred.

When used herein "consisting of" excludes any element, step, or ingredient not specified in the claim element. When used herein, "consisting essentially of"" does not exclude materials or steps that do not materially affect the basic and novel characteristics of the claim.

The term "disease" as used herein is intended to be generally synonymous, and is used interchangeably with, the terms "pathology", "disorder" and "condition" (as in medical condition), in that all reflect an abnormal condition of the body or of one of its parts that impairs normal functioning and is typically manifested by distinguishing signs and symptoms.

A "therapeutically effective amount" as referred herein is defined as an amount of a compound that, when administered to a subject, is sufficient to effect such treatment of a conditioned state. The "therapeutically effective amount" may vary depending on the compound, and condition being treated, the age and relative health of the subject, the route and form of administration, the judgment of the one having ordinary skill in the art, and other factors.

The term "treatment" as used herein refers to the medical care given to a patient for a disease or injury. This term includes curing the disease but also ameliorating, mitigating, or reducing the symptoms of said disease. Thus, the term "therapeutic treatment" or "treatment" as used herein refers to bringing a body from a pathological state or disease back to its normal, healthy state. The term "therapeutic treatment" or "treatment" as used herein, also refers to the mitigation, amelioration, or reduction of the harmful effect of radiation in a subject, or in some manner reducing the symptoms associated with such effects. The term "prophylactic treatment" as used herein refers to preventing a pathological state.

With "prevention" is meant keeping the disease from happening. With "amelioration" is meant an improvement in a patient's disease, or the activity of making an effort to correct, or at least make more acceptable said disease. With "reduction" or "mitigation" is meant decreasing the severity, seriousness or painfulness of the disease.

The term "subject" as used herein refers to a mammalian subject. Preferably, it is selected from a human, companion animal, non-domestic livestock or zoo animal. For example, the subject may be selected from a human, dog, cat, cow, pig, sheep, horse, bear, and so on. In a preferred embodiment, said mammalian subject is a human subject.

With "ionizing radiation" is meant the radiation consisting of particles, X-rays, or gamma rays with sufficient energy to cause ionization in the medium through which it passes. With "γ rays" or "γ radiation" is meant a penetrating form of electromagnetic radiation arising from the radioactive decay of atomic nuclei. It consists of the shortest wavelength electromagnetic waves and so imparts the highest photon energy. With "X-rays" or X radiation is meant or X-radiation, is a penetrating form of high-energy electromagnetic radiation. Most X-rays have a wavelength ranging from 10 picometers to 10 nanometers, corresponding to frequencies in the range 30 petahertz to 30 exahertz and energies in the range 124 eV to 124 keV.

With "Pterostilbene" or PT is meant 3,5-dimethoxy-4'-hydroxystilbene or a salt or derivative thereof. Pterostilbene (PT) is a natural antioxidant and a natural analogue of Resveratrol, but almost 60-100 times more potent antifungal agent that shows similar anticarcinogenic properties. It is present predominantly in blueberries and red grapes, although quantitative studies show that for every 10 parts of t-Resveratrol, there are only 1-2 parts of t-Pterostilbene. Pterostilbene corresponds to the formula:

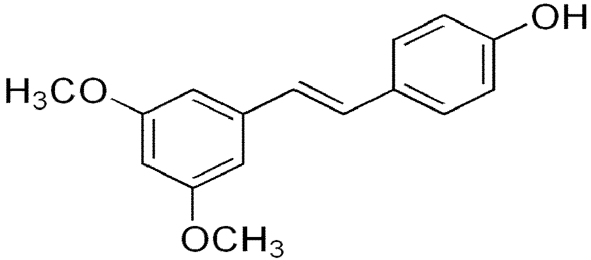

With "Silibinin" or SIL also known as silybin (both from Silybum (milk thistle) is a flavonoid obtain from a genus of two species of thistles in the daisy family. The plants are native to the Mediterranean regions of Europe, North Africa, and the Middle East. Silibinin (SIL), is the major active constituent of silymarin, a standardized extract of the milk thistle seeds, containing a mixture of flavonolignans consisting of silibinin, isosilibinin, silichristin, silidianin, and others. Silibinin itself is a mixture of two diastereomers, silybin A and silybin B, in approximately equimolar ratio. SIL is also known as 3,5,7-trihydroxy-2-[3-(4-hydroxy-3-methoxyphenyl)-2-(hydroxymethyl)-2,3-dihydro-1,4-benzodioxin-6-yl]-2,3-dihydrochromen-4-one with the following formula: 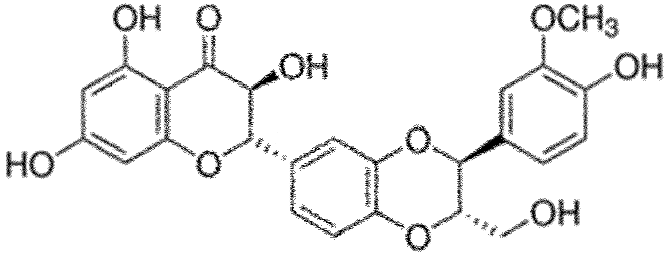 By Nicotinamide adenine dinucleotide booster, NAD+ precursors or NAD+ boosters is meant any compound as clearly disclosed on the document by Rajman et al., Cell Metab. 2018 March 06; 27(3): 529-547. doi:10.1016/j.cmet.2018.02.011, such as NAD precursors, activators of NAD synthesis, or inhibitors of NAD+ degradation.

By "Fibroblast-Stimulating Lipopeptide" or FSL-1 is meant the Toll-Like Receptor 2/6 Agonist, or salt of derivatives thereof, and corresponds to the formula:

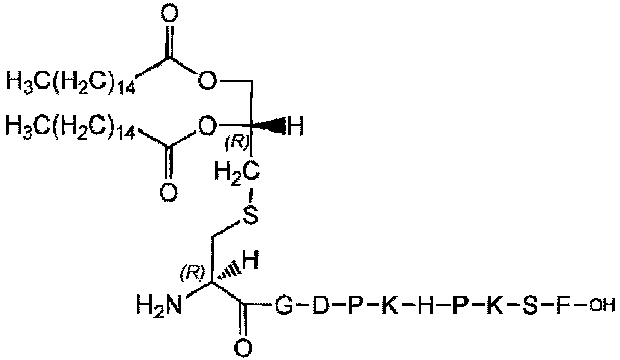

By Nicorinamide riboside is meant a pyridine-nucleoside similar to vitamin B3, functioning as a precursor to nicotinamide adenine dinucleotide or NAD+. The formula of NR is:

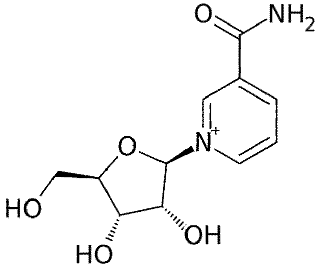

The term "kit of parts" as used herein refers to a combined preparation wherein the active ingredients are physically separated for use in a combined therapy by simultaneous administration or sequential administration to the patient.

### DETAILED DESCRIPTION

Several natural compounds are known to have radioprotective effects when administered to a subject before radiation. As shown in Figure 2A, compounds such as pterostilbene (PT), gallic acid, EGCG, etc. provide around 100% survival 6 days after radiation in Swiss albino mice. However, some compounds fail to protect the mice for longer periods of time. For instance, after 30 days (Figure 2B), the percentage of survival of Luteolin was reduced in about 40%, and after 60 days (Figure 2C), its percentage of survival was 0%. Thus, compounds able to exert high level of protection during prolonged period of times are desirable.

Although these compounds can be combined and administered together, the radioprotection resulted from this combination cannot be predicted. In some cases, the radioprotective properties of one compound are not altered when this compound is combined with another compound. For instance, as shown in Figure 2C, the administration of PT produces 40% mice survival 60 days after application of radiation. Delphinidin provides around 12% survival. However, when PT is combined with Delphinidin, the resulting survival remains around 40%, which is approximately the survival provided by the PT compound alone. In this case, the effect of Delphinidin is not added to the effect of PT.

In other cases, surprisingly, the radioprotective effect of a compound is negatively affected by the co-administration of another compound. This is shown in Figure 2C, where the radioprotective effect of PT provides a 40% survival after 60 days, and Gallic acid provides around 5% protection. However, when both compounds are combined, the overall rate of survival is around 35%, resulting in a reduced radioprotective effect than the administration of PT alone.

In other cases, the radioprotective effect of two compounds is summed when they are combined, resulting in a protection derived of their individual effects added together. For instance, in Figure 2C it is shown that the protection provided by PT is around 40%, and the protection provided by Genistein is around 7%. Thus, when both compounds are combined, the resulting protection amounts to around 45%, which means that both compounds are exerting their effect and the overall protection is, more or less, the sum of the effects of both compounds.

More interestingly, the authors of the present invention have surprisingly found that, not only the combination of two compounds can result in no changes, or detrimental, or accumulative protection, but also, in very few cases cases, the combination of two compounds results in enhanced radioprotective properties that go beyond the mere accumulation of their effect. That is, the specific combinations of specific compounds provide an enhanced protection that cannot be explain by the accumulation of their individual effects, which means that there is a synergistic effect among them. This is shown in Figure 2B and 2C. In figure 2C, Silibinin (SIL) provides around 10% survival and PT provides around 40% survival, but the combination of both of them provides more than 70% survival. This clearly shows that the combination of SIL and PT does not merely produce an added effect of their radioprotective properties, but also produces a synergistic effect resulting in an enhanced radioprotection.

These findings suggest that, to obtain enhanced radioprotection, care should be taken with the compounds that are combined, since some combinations are able to enhance the radioprotection, while other combinations are detrimental, and in this later case it would be more advisable to administrate the compounds individually.

Thus, in order to obtain an enhanced radioprotection, one cannot just arbitrarily combine compounds that have different radioprotective properties, since this combination does not always result in an overall enhanced radioprotection. Depending on which compounds are co-administrated, the resulting radioprotection can be worse, can be the same or the sum of the two compounds, but that also, some compounds combined together provide with a synergistic effect and an enhanced protection that is not derivable from the mere sum of their radioprotective properties.

Another aspect that was found by the inventors is that the specific combination of PT and SIL provide protection for long periods of time. This is surprising because, as stated above, not all compounds exert a radioprotective effect for more than 30 days, see, for instance, EGCG, Quercetin, Naringerin or Phloridzin compounds in Figure 2B (30 days), where their radioprotective effect is reduced to 0% after 60 days (Figure 2C). However, as shown in Figure 3, the combination of PT and SIL provided around 25% survival (5 mice alive out of 20) at day 360, that is, after a year. Thus, the combination of PT and SIL not only results in a synergistic enhanced protective effect, but this effect is also durable. Additionally, as shown in Figure 11B, when PT and SIL are combined with other radiomitigator compounds, such as nicotinamide riboside (NR) or fibroblast-stimulating lipopeptide-1 (FSL-1), the survival is even further increased to up to 50-60% after a year and, remarkably, when the four compounds (PT, SIL, NR and FSL-1) are combined, they provide a nearly full protection (90%) of the animals after 360 days.

In conclusion, the present invention provides two main findings: on the one hand, it was found that the co-administration of PT and SIL, two natural compounds with radioprotective properties, provide an enhanced and durable synergistic radioprotection not derived from the mere accumulation of their corresponding radioprotective effects. On the other hand, it was also found that the combination of these two compounds with a radiomitigator compound, such as NR and/or FSL-1, can further enhance the protection to radiation up to 90% of survival for long period of times, even for a year.

### Pterostilbene for use in the prevention, amelioration or reduction of diseases induced by ionizing radiation.

A **first aspect** of the present invention relates to Pterostilbene, or a pharmaceutically acceptable salt thereof, for use in the treatment, prevention, amelioration or reduction of diseases induced by ionizing radiation in a subject, in combination with Silibinin, or a pharmaceutically acceptable salt thereof, wherein the administration is carried out before the radiation, or after the radiation, or during the radiation; and wherein the combination is herein understood as the simultaneous or sequential administration, in any order, of a) Pterostilbene, or a pharmaceutically acceptable salt thereof, and b) Silibinin, or a pharmaceutically acceptable salt thereof, to said subject.

In a particular embodiment, the diseases induced by ionizing radiation are related to DNA damage, radiation-induced cytotoxicity, genotoxicity and/or oxidative cellular damage. In a preferred embodiment, the diseases induced by the ionizing radiation are acute radiation syndrome and/or chronic radiation syndrome. In another embodiment, said diseases are selected from the group consisting of skin diseases, acute radiation syndrome, cancer and cardiovascular diseases.

Under the first aspect of the present invention, Pterostilbene is used in combination with Silibinin. PT and SIL may be administered in a sole composition or in separate compositions.

In a preferred embodiment, the Pterostilbene is Pterostilbene phosphate disodium salt. In another preferred embodiment, the Silibinin is silibinin-C-2',3-dihydrogen succinate, disodium salt.

As stated above, PT and SIL, or any pharmaceutically acceptable salts or derivatives thereof, are administered in combination to prevent, ameliorate or reduce diseases induced by ionizing radiation in a subject. In a preferred embodiment, the subject is a mammal. More preferably, the subject is a human being.

### Administration schedule and route

The administration can be simultaneous (at the same time) or sequential. "Sequential administration" as used herein refers to the administration of SIL or a composition comprising thereof shortly before or after administration of PT or a composition comprising thereof. "Shortly before or after" as used herein is understood as within an interval of 1 to 24 hours, preferably 1 to 12 hours, preferably 1 to 3 hours, preferably within 2 hours, more preferably within 1 hour or less, such as within 45 minutes, 30 minutes, 15 minutes or less. In an embodiment, PT is administered first followed by SIL. In another embodiment, SIL is administered first followed by PT. In a preferred embodiment, PT and SIL administration is carried out simultaneously. In an embodiment, the PT and SIL are administered in combination simultaneously, as a part of a single composition or as part of two different compositions.

In an embodiment, said pharmaceutically acceptable compounds and compositions are formulated to be compatible with its intended route of administration. Methods to accomplish the administration are known to those of ordinary skill in the art. This includes, for example, injections, by parenteral routes such as intravascular, intravenous, intraarterial, subcutaneous, intramuscular, intraperitoneal, intraventricular, intraepidural, or others as well as oral, topical, nasal, ophthalmic, rectal, or topical. Sustained release administration is also specifically contemplated, by such means as depot injections or erodible implants. A preferred route of administration is parenteral administration, which is herein understood as the administration within a vessel or vessels and typically includes intravenous or intraarterial administration. Currently, the most preferred route of administration is parenteral, oral or topical.

The administration can be carried out only once, or there can be more than one administration. In a preferred embodiment, there compounds are administered several times. In a particular embodiment, the administration may comprise at least one, two, three, four, five, six, seven or more doses.

The administration can be performed before, during or after the radiation occurs. For example, the administration can be performed at least 10, 9, 8, 7, 6, 5, 4, 3, 2, 1 days before the radiation occurs. In another embodiment, the administration occurs after radiation. In an embodiment, the administration carried out after the radiation occurs 1, 2, 3, 4, 5, 6, 7, 8, 9, 10 or more than 10 days, such as 20 or 30 days, or more than one moth, such as 2, 3, 5, 6, 8, 9 months after radiation. In a particular embodiment, the administration is carried out on the same day where the radiation occurs, either before or after it. In an embodiment, the administration consists of several doses administered during several days before, on the same day, and/or after the radiation occurs. In a preferred embodiment, Pterostilbene and Silibinin, or any pharmaceutically acceptable salts thereof, are administrated several times simultaneously or sequentially before and/or after radiation, or both.

### Dosage

The optimal dose will be selected according to the administration route, treatment regime and/or administration schedule, having regard to the existing toxicity and effectiveness data. In a preferred embodiment, PT and SIL for use according to the first aspect of the present invention are administered in a dosage capable of providing a radioprotective effect. As defined above, "radioprotection" is understood the ability to reduce the direct damage caused by radiation. Accordingly, in the present invention, the dosage of PT and SIL is not particularly restricted.

In in an embodiment, PT, or a pharmaceutically acceptable salt thereof, is administered at a dosage up to 500 mg/kg. PT may be administered at dosages ranging from 300 mg/kg to 30 mg/kg. Preferably, PT is administered at a dosage from 200 mg/kg to 40 mg/kg or 150 mg/kg to 40 mg/kg. More preferably, PT is administered at a dosage of 100 mg/kg.

In another embodiment, SL, or a pharmaceutically acceptable salt thereof, is administered at a dosage up to 500 mg/kg. SIL may be administered at a dosage ranging from 200 mg/kg to 35 mg/kg. Preferably, SIL is administered at a dosage from 150 mg/kg to 40 mg/kg or 100 mg/kg to 50 mg/kg. More preferably, PT is administered at a dosage of 70 mg/kg.

### Administration of further compounds

In an embodiment of the first aspect, the use described in any of the above mentioned embodiments further comprises the administration in combination with at least one further radiomitigator compound, wherein the use described herein is herein understood as the simultaneous or sequential administration of a) Pterostilbene, or a pharmaceutically acceptable salt thereof, b) Silibinin, or a pharmaceutically acceptable salt thereof, and c) one or more radiomitigator compounds, to said subject. In a preferred embodiment, the at least one or more radiomitigator compound/s refers to a nicotinamide adenine dinucleotide booster (referred from now on as NAD+ boosters), and/or a Fibroblast-Stimulating Lipopeptide-1 (referred from now on as FSL-1), or any pharmaceutically acceptable salts thereof. Please note that, in the context of the present invention, although FSL-1 is preferred, FSL-1 is a type of Fibroblast-Stimulating Lipopeptide and can be thus replaced by any other Fibroblast-Stimulating Lipopeptide. Consequently, the term "FSL-1" as used in any of the embodiments of the present invention can be thus replaced by any other suitable Fibroblast-Stimulating.

As defined above, a "radiomitigator" is a compound that is able to minimize toxicity after even after radiation has been delivered. Thus, in an embodiment of the first aspect, the administration of PT combined with SIL further comprises the administration of one or more radiomitigator compound/s. The combinations between PT, SIL and the one or more radiomitigator compound/s are not limited, that is, PT can be administered in a single composition together with SIL and one or more radiomitigator/s, or they can be administered individually in different compositions. All the possible combinations of the three (or four or more) compounds are included herein.

The administration routes and schedules described above also apply in the case of PT and SIL administered together with one or more radiomitigator compound/s.

In a preferred embodiment, the use of PT in the prevention, amelioration or reduction of diseases induced by ionizing radiation in a subject, in combination with SIL, their salts or derivative thereof, further comprises the administration of a NAD+ booster. Thus, in this embodiment, the at least three compounds are administered together, and can be comprised in the same or in different compositions and can be administered sequentially or simultaneously.

In another preferred embodiment, the use of PT in the prevention, amelioration or reduction of diseases induced by ionizing radiation in a subject, in combination with SIL, their salts or derivative thereof, further comprises the administration of FSL-1. Thus, in this embodiment, the at least three compounds are administered together, and can be comprised in the same or in different compositions and can be administered sequentially or simultaneously.

In an even more preferred embodiment, the use of PT in the prevention, amelioration or reduction of diseases induced by ionizing radiation in a subject, in combination with SIL, their salts or derivative thereof, further comprises the administration of both a NAD+ booster and FSL-1. Thus, in this embodiment, the four compounds are and can be comprised in the same or in different compositions and can be administered sequentially or simultaneously.

In a more preferred embodiment, the NAD+ booster is Nicotinamide riboside or 1-(β-D-Ribofuranosyl)nicotinamide (referred from now on as NR), a pharmaceutically acceptable salt or a derivative thereof.

In an even more preferred embodiment, the use of PT in the prevention, amelioration or reduction of diseases induced by ionizing radiation in a subject, in combination with SIL, their salts or derivative thereof, further comprises the administration of NR and FSL-1. Thus, in this embodiment, the four compounds are administered together, in one or more than one doses, comprised in the same or in different compositions, and administered sequentially or simultaneously before, during or after the radiation.

In a preferred embodiment, PT and SIL are administered once per day during several days before the radiation and/or several days after the radiation. More preferably, PT and SIL are administered daily during 10, 9, 8, 7, 6, 5, 4, 3, 2, 1, preferably 5, days before the radiation. In another embodiment, PT and SIL are administered daily before the radiation and up to 5, 4, 3, 2, 1 days after the radiation. Preferably, PT and SIL are administered daily during the 5 previous days before the radiation and during the 2 days after radiation.

In another embodiment, the NAD+ booster is administered once per day on the same day of the radiation and during the next month after radiation. In another embodiment, the NAD+ booster is administered at least 60 mins after the radiation and it is further administered during the next 30 days after the radiation.

In another embodiment, the FSL-1 is administered only once, preferably after the radiation, more preferably at least one day after the radiation.

In an embodiment, the NAD+ booster, or a pharmaceutically acceptable salt thereof, is administered at a dosage up to 500 mg/kg. In an embodiment, the NAD+ booster, or a pharmaceutically acceptable salt thereof, is administered at a dosage from 400 mg/kg to 100mg/kg. Preferably, the NAD+ booster is administered at a dosage from 300 mg/kg to 150 mg/kg or 200 mg/kg to 150 mg/kg. More preferably, the NAD+ booster is administered at a dosage of 185 mg/kg.

In another embodiment, the NAD+ booster is Nicotinamide Riboside, or any pharmaceutically acceptable salt thereof, and it is administered at a dosage from 400 mg/kg to 100mg/kg. Preferably, the Nicotinamide Riboside is administered at a dosage from 300 mg/kg to 150 mg/kg or 200 mg/kg to 150 mg/kg. More preferably, the Nicotinamide Riboside is administered at a dosage of 185 mg/kg.

In in an embodiment, FSL-1, or a pharmaceutically acceptable salt thereof, is administered at a dosage up to 1mg/kg. In another embodiment, the FSL-1 compound, or a pharmaceutically acceptable salt thereof, is administered at a dosage from 1 mg/kg to 0,1 mg/kg. Preferably, the FSL-1 compound is administered at a dosage from 0.75 mg/kg to 0.1 mg/kg or 0.5 mg/kg to 0.1 mg/kg. More preferably, FSL-1 compound is administered at a dosage of 0.25 mg/kg.

In an embodiment, the ionizing radiation is gamma (γ) rays or X-rays.

### A composition comprising PT and SIL

In a **second aspect,** the invention relates to a pharmaceutical composition comprising a) Pterostilbene, or a pharmaceutically acceptable salt thereof, and b) Silibinin, or a pharmaceutically acceptable salt thereof.

Pharmaceutically acceptable excipients include, but are not limited to, liquids such as water, saline, polyethyleneglycol, hyaluronic acid, glycerol and ethanol. Pharmaceutically acceptable salts can also be included therein, for example, mineral acid salts such as hydrochlorides, hydrobromides, phosphates, sulfates, and the like; and the salts of organic acids such as acetates, propionates, malonates, benzoates, and the like. It is also preferred, that the preparation will contain a pharmaceutically acceptable excipient that serves as a stabilizer, particularly for peptide, protein or other like molecules if they are to be included in the vaccine composition. Examples of suitable carriers include, without limitation, pharmaceutical grades of dextrose, sucrose, lactose, trehalose, mannitol, sorbitol, inositol, dextran, and the like. Other suitable carriers include, starch, cellulose, sodium or calcium phosphates, citric acid, tartaric acid, glycine, high molecular weight polyethylene glycols (PEGs), and combination thereof. A thorough discussion of pharmaceutically acceptable excipients, additives and carriers is available in Remington's Pharmaceutical Sciences (22nd edition, 2012).

Said pharmaceutical composition or compositions, comprise PT and SIL in the dosages defined in the first aspect of the invention and it is administered according to the administration route and schedules defined under the first aspect of the invention. Further details and preferred embodiments on the dosage, route and schedules of administration of said composition are also provided under the first aspect of the invention.

Said pharmaceutical composition comprise PT and SIL, or any pharmaceutically acceptable salts or derivatives thereof, and may further comprise one or more radiomitigator compound/s, as defined under the first aspect of the invention. As stated above, in an embodiment, the radiomitigator compound may be NAD+ booster and/or FSL-1. In a preferred embodiment, the composition comprising PT and SIL further comprises at least two radiomitigator compounds, preferably NR and FSL-1.

Preferably, said composition or compositions is in a form suitable for parenteral, oral or topic administration. In a preferred embodiment, said composition is an aqueous composition, more preferably a stable aqueous composition. As used herein, a "stable composition" may refer to a formulation in which the active ingredient therein essentially retains its physical stability and/or chemical stability and/or biological activity upon storage.

The present invention further relates to said composition for use as defined in the first aspect of the invention.

### A kit of parts comprising PT and SIL

In a **third aspect,** the present invention relates to kit of parts comprising at least two recipients comprising at least Pterostilbene and Silibinin, or any pharmaceutically acceptable salts thereof, and optionally at least one or more radiomitigator compounds as defined above.

Typically, the two or more active ingredients are provided formulated as a pharmaceutical composition and provided in two or more separate recipients. For instance, PT and SIL can be combined in one recipient, and the radiomitigators in the other. In an embodiment, each compound is contained in a separate recipient. In further embodiments, the radiomitigator compounds are present in similar recipients as the radiomitigator compounds.

Preferably, said compositions are as defined in the previous aspects of the invention, for use as defined in the previous aspects of the invention, preferably in the therapeutic treatment of a disease induced by ionizing radiation.

### Method for preventing, ameliorating or reducing diseases induced by ionizing radiation

In a **fourth aspect,** the present invention relates to a method for preventing, treating, including ameliorating or reducing, diseases induced by ionizing radiation in a subject, comprising administering Pterostilbene in combination with Silibinin, or any pharmaceutically acceptable salts thereof, wherein said administration is performed before the radiation, or after the radiation, or during the radiation; and wherein the combination is herein understood as the simultaneous or sequential administration of a) Pterostilbene, or a pharmaceutically acceptable salt thereof, b) Silibinin, or any pharmaceutically acceptable salt thereof, and optionally, c) at least one or more radiomitigator compounds, as defined throughout the present specification, to said subject. Similar as described above in the first and second aspects of the present invention, the at least one or more radiomitigator compounds may be NAD+ boosters and/or FSL-1.

This fourth aspect is related to the first and second aspects described above. Thus, the compounds and the pharmaceutical compositions, administration dosages, routes and schedules are provided under the previous aspects of the invention.

It will be understood that particular embodiments described in the examples are shown by way of illustration and not as limitations of the invention. The principal features of this invention can be employed in various embodiments without departing from the scope of the invention. Those skilled in the art will recognize or be able to ascertain using no more than routine experimentation, numerous equivalents to the specific procedures described herein. Such equivalents are considered to be within the scope of this invention and are covered by the claims.

### EXAMPLES

### EXAMPLE 1: General Materials and Methods

Disclosed herein are general materials and methods used in the present invention. Particular procedures are further described in each of the Examples 2-4.

### Mice and treatment procedures

Swiss albino mice (male, 9-10 weeks old, 30-32 g) were obtained from Charles River Laboratories (Wilmington, MA). This strain is among the most common used in radiation studies (Williams 2010). Mice were fed with a nutritionally complete diet (standard diet, SD) before irradiation. This was A03 from Panlab (Barcelona, Spain), which has 3,200 kcal/kg and the following nutritional composition: crude protein, 23.5%; crude fat, 4.3%; crude fiber, 3.7%; and carbohydrates, 51.1%; vitamins and minerals were provided as in the NIH-7 open formula for rat and mouse. This diet meets the dietary allowances recommended for adult mice by the American Institute of Nutrition. The animals were fed *ad libitum* and kept in individual metabolic cages, in which body weight and food ingestion were monitored daily. The animal room was maintained at 22°C on a 12 h light/12 h dark cycle. *Adlibitum* intake of drinking water was also allowed. Care of irradiated mice followed the recommendation of the Robert Wood Johnson Medical School (Piscataway, **NJ)** for mouse total body irradiation (policy 15), which includes: a) use of antibiotics in the drinking water (134 mg ampicillin/kg/day, 40 mg baytril/kg/day, and 220/42 mg sulfamethoxazole/trimethoprim/kg/day), b) making drinking water readibly available, c) Napa nectar Systems Engineering Lab Group Inc., Napa, CA) provided on the bottom of the cage during the first 14 days and replaced daily , d) provision of softened food served in a small Petri dish on the cage floor, e) completely sterile environment (cage, food, and water) to avoid potential immunosuppression-associated infections. A freestanding wheel inside the cage was also included to facilitate the physical exercise of the animals. All experiments were started at 10.00 h to minimize possible circadian variations. Procedures involving animals were in compliance with international laws and policies (EEC Directive 86/609, OJ L 358. 1, December 12, 1987; and NIH Guide for the Care and Use of Laboratory Animals, NIH Publ. No. 85-23, 1985). Mice were subjected to g-radiation (¹³⁷Cs) (total body irradiation, TBI) in a BIOBEAM 8000 (Gamma-Service Medical GmbH, Leipzig, Germany). Single fraction radiotherapy, at total doses ranging from approx. 5.0 to 10.0 Gy, was administered at a rate of approx. 5.0 Gy/min. The irradiation distance from the source was limited to 12 cm. Radiation dosimetry was controlled using alanine dosimeters from Bruker (Billerica, MA) with dosimetry variation of +/- 0.09 Gy. For oral administration PTER and SIL (Merck Chemicals GmbH, Darmstadt, Germany) were solubilized in 2-hydroxypropyl-b-cyclodextrin, then suspended in 0.3% carboxymethylcellulose. For IP administration, a PTER salt (pterostilbene phosphate disodium salt, SYNCOM, Groningen, The Netherlands) and SIL salt (silibinin-C-2',3-dihydrogen succinate, disodium salt, Rottapharm/Madaus, Cologne, Germany) were dissolved in sterile vaccination-grade water. All other polyphenolic phytochemicals assayed (Merck Chemicals GmbH; and Cayman Chemical Co., Ann Arbor, MI) represent main flavonoids and non-flavonoids (Estrela 2017), i.e. gallic acid (a phenolic acid), caffeic acid (a hydroxycinnamic acid), curcumin (a curcuminoid), epigallocatechin gallate (a flavanol), genistein (a isoflavone), quercetin (a flavonol), luteolin (a flavone), naringenin (a flavanone), delphindin (an anthocyanidin), and phloridzin (a chalcone) were solubilized as PTER and SIL before oral administration (Wani 2016). NR (nicotinamide riboside, 3-aminocarbonyl-1-b-D-ribofuranosyl-pyridinium, Elysium Health, Inc., New York, NY) was dissolved in vaccination-grade water, and the pH of the solution neutralized (pH 7.0) before oral administration (185 mg/kg x day, Obrador 2020). FSL-1 (InvivoGen, San Diego, CA) was resuspended in sterile vaccination-grade water, and administered IP (0.25 mg/kg x day, the volume administered was of 50-60 mL depending on the weight of the mouse, as recommended by Kurkjian 2017). Pharmaceutical grade amifostine (Ethyol) was obtained from Cumberland Pharmaceuticals (Nashville, TN) as 500 mg sterile lyophilized powder vial, and reconstituted with physiological saline prior to use.

### Pathology

Mice were removed from the study either dead, moribund, or scheduled for sacrifice. All mice were necropsied, and approx. 30 tissues or organs as well as all gross lesions were collected for microscopic examination. After fixation in 10% neutral-buffered formalin, all tissues were processed routinely and stained with hematoxylin and eosin for histopathological evaluation.

### Rotarod test

Functions of the test include evaluating balance, grip strength and motor coordination of the subjects; especially in testing the effect of experimental drugs. For this test (Rota Rod, Harvard Apparatus, Holliston, MA) each animal was given three trials and the maximum period (seconds) that it could remain on a rotating axle (3.5 cm diameter; speed of rotation: 15 rpm) without falling was measured. Each mouse was given up to three attempts for an arbitrary limit of 1200 seconds and the longest period was recorded. Changes over time in the different groups were monitored weekly.

### Pterostilbene levels

Liquid chromatography and mass spectrometry (LC-MS/ MS) was performed, as previously described (Ferrer 2005), using a TSQ Vantage^{™} Triple Quadrupole Mass Spectrometer (Thermo Fisher Scientific) equipped with a Shimadzu LC-10ADvp. pump and an SLC-10Avp. controller system with an SIL-10ADvp. autoinjector.

### Silibinin levels

Measurement was based on the methodology described by Song et al. (2019). Briefly, samples were analyzed through HPLC (Ultimate 3000, Thermo Fisher Scientific) and MS/MS (Waters TQ detector triple quadrupole) systems on a Hypurity^{™} C18 column (50 × 4.6 mm) using an injection volume of 5 µL. The mobile phases consisted of water (A) and acetonitrile (B). The gradient elution system was optimized as follows: 0-3.0 min 20% - 100% B, 3.0-5.0 min 100% - 100% B, 5.0-5.5 min 100% - 20% B, 5.5-7.5 min 20% B. The flow rate was 0.5 mL/min. MS/MS data acquisition was performed under negative electrospray ionization mode. The multiple reaction monitoring mode was employed to monitor SIL with the precursor-to-product ion transition of m/z 481.3/125.0. The fragmentary voltage was 150 V, and the collision energy 15 eV. Tissue sample preparation was as described for determination of PTER levels (above).

### Blood NAD⁺ levels

Measurement of NAD⁺ levels in total blood was performed by HPLC following the methodology described by Yoshino and Imai (...). For this procedure we used an HPLC system (Shimadzu, Kyoto, Japan) with a Supelco LC-18-T column (15 cm × 4.6 cm; Sigma Aldrich) and a Hypercarb column (15 cm × 4.6 cm; Thermo Fisher Scientific, Waltham, MA), respectively. Whole-blood samples (0.5 ml) were collected in sodium citrate tubes at 4°C. Then a whole-blood aliquot of 0.1 ml was transferred to a cryovial containing 0.9 ml of 0.5 M ice-cold perchloric acid to lyse all blood cells. Samples were centrifuged at 10,000 g × 10 min (4°C). Supernatants were stored at -80°C until analyzed.

### Evaluation of therapy-induced in vivo toxicity

This included blood cell count and chemistry based on NIH standard methodology.

### Chromosomal aberrations and double-strand DNA breaks

We have followed the technique described by M'kacher et al. (2015) where telomere and centromere staining facilitates the easy detection of dicentrics, centric rings, and acentric chromosomes of premature chromosome condensation in lymphocytes using optimized hybridization conditions and fusion capture of interphase chromosomes. This approach presents a major advantage since damage can be observed within hours after blood sampling. Blood was collected 8 h after irradiation as suggested in the procedure (M'kacher 2015), thus allowing time for DNA repair and the occurrence of dicentrics, centric rings, and acentric chromosomes

Peripheral blood lymphocytes were isolated as described here below. Premature chromosome condensation (PCC) was performed as previously described (Pantelias 1983) using CHO cells (ATCC). The condensed human chromosomes were distinguished from the CHO chromosomes according to their morphologic characteristics (M'kacher 2015). This procedure required approx. 4 h.

Telomeres and centromeres of PCC fusions were stained using the Q-FISH technique with a Cy-3-labeled peptide nucleic acid (PNA) probe specific for TTAGGG of telomere and a fluoroscien isothiocyanate-labeled PNA probe specific for centromere sequences (M'kacher 2015) (both from ThermoFisher Scientific, Waltham. MA). Telomere and centromere-stained fusions were automatically recorded using the Autocapt software from Metasystems (Altlussheim, Germany) and a high resolution CCD camera (C91002-23B, Hamamatsu Photonics, Herrsching, Germany).

In the same slide two different manual procedures were performed as described by M'kacher et al. (2015). Briefly, first using the reverse 4',6-diamidino-2-phenylindole, and scoring rings and excess acentric chromosomes. Second using the telomeres and centromeres staining to score dicentrics, centric rings, and acentric chromosomes from interstitial deletions without telomere staining, acentric chromosomes with only 1 telomere, representing terminal deletions, and acentric chromosomes with 2 telomeres derived from the fusion of 2 acentric chromosomes accompanying the formation of dicentrics or acentric rings. Resulting double-strand DNA breaks (DSBs) were calculated (M'kacher 2015).

### Micronuclei formation

For this purpose the femoral bone marrow was collected from sacrificed mice, and smear was prepared and fixed using methanol. After drying, double staining was performed using May Grunwald and Giemsa stains (Schmid 1975). According to this method, polychromatic erythrocytes (PCE) stained reddish-blue and normochromatic erythrocytes (NCE) stained orange, while nuclear materials were dark purple. The micronucleated polychromatic (MnPCE) and normochromatic (MnNCE) cells were counted out of 2000 cell under oil immersion.

### Isolation and incubation of lymphocytes, hepatocytes, and intestinal epithelial cells

Isolation of hepatocytes was performed according to previously reported methodology (Berry 1969). Blood mononuclear cells were obtained from heparinized blood by Accuspin-Histopaque (Sigma-Aldrich, St. Louis, MO) gradient centrifugation. Mononuclear cells were washed twice and resuspended in Krebs-Henseleit bicarbonate medium (pH 7.4) with 0.3% bovine serum albumin at a concentration of 20 × 10⁶ cells/ml. Further positive selection of lymphocyte subsets by monodispersed immunomagnetic Dynabeads (Dynal) was performed at 4°C, as described elsewhere (Thornton 2003). Hepatocyte and lymphocyte cell volumes were obtained as previously described (Estrela 1992).

Isolated hepatocytes were incubated in flasks (about 10 mg dry wt/ml) at 37°C in Krebs-Henseleit bicarbonate medium (KHBM, pH 7.4) containing 1.3mM-CaCl₂, 5%fat-free bovine serum albumin, and in the presence of glucose (5mM). The gas atmosphere was O₂/CO₂ (19:1). Isolated lymphocytes were incubated (10⁶ cells/flask) at 37°C in Krebs-Ringer medium with 5% fat-free bovine serum albumin and in the presence of glucose (5mM) and glutamine (2 mM). After incubation, the cells were disrupted by the addition of 0.2 ml of 25% perchloric acid. Protein was removed by centrifugation and the supernatant was neutralized with 20 ml of a 40% KOH solution and a Tris-(hydroxymethyl) aminomethane/KOH (0.5-2.0 M) solution for the measurement of the metabolites.

Primary intestinal epithelial cells (IECs) were isolated using a protocol adapted from Graves et al. (2014). Colon tissue was prepared by removing the longitudinal muscle layer and washing with ice-cold Mg²⁺- and Ca²⁺-free Hank's Balanced Salt Solution (HBSS) containing 100 U penicillin, 100 pg/ml streptomycin, 25 pg/ml gentamycin and 0.5 mM dithiothreitol (DTT). Tissue was cut into small pieces, suspended in 50 ml HBSS wash solution and shaken, and the contents were allowed to settle for 2 min. The supernatant was removed and the settled contents were washed 3 times. The settled contents were minced and suspended in HBSS. The resulting suspension was passed over a 1000 µm² mesh filter. Remaining tissue was digested in a buffer containing 75 U/ml collagenase type XI (Sigma-Aldrich), 25 pg/ml dispase neutral protease II (ThermoFisher Scientific, Waltham, MA), 0.5 mM DTT, and 2% v/v fetal bovine serum (FBS) (ThermoFisher Scientific) in Dulbecco's Modification of Eagles Medium (DMEM) (Sigma-Aldrich). The digestion buffer containing the tissue was then placed in a 37 °C incubator and allowed to shake at 200 rpm for 2 h. The resulting digestion mixture was again passed over a 1000 µm2 filter, and the tissue fragments atop the filter were washed with 25 ml complete growth media (DMEM, 8.5 g/l sodium pyruvate, 2% v/v FBS, 0.25 U/ml insulin, 100 U penicillin, 100 pg/ml streptomycin, 25 µg/ml gentamycin, 5 pg/ml transferrin, and 10 ng/ml EGF containing 2% w/v D-sorbitol. Tissue debris remaining in the filter was discarded, and the effluent containing proliferative crypt structures was centrifuged at 200g, for 5 min, at 4 °C. The remaining pellet containing isolated intestinal crypts was suspended in DMEM. The crypts were suspended in the complete growth media, plated at a density of approximately 2000 crypts/ml/well in a 12-well type I collagen-coated culture dish (ThermoFisher Scientific) and incubated at 37 °C, O₂/CO₂ (19:1).

Rates of glucose and glutamine utilization were measured as previously described (Newsholme 1987).

### Cell culture

Isolated hepatocytes were cultured in William's complete medium at a concentration of 10⁶ cells/mL. Isolated C2D⁺ lymphocytes were cultured in PB-MAX medium (GIBCO, ThermoFisher Scientific) at a concentration of 2 × 10⁶ cells/mL. IECs (10⁶ cells/mL) were cultured in DMEM supplemented with 10% heat-inactivated FBS, and 300 mg/ml L-glutamine. All cells were cultured at 37°C in a humidified atmosphere of 95% air and 5% CO₂.

### DNA oxidation

Tissue samples were homogenized in 10 mM Tris (pH 7.0) +1 mM EDTA + 150 mM NaCl. Thereafter they were incubated first with RNase A at 37 °C for 30 min and then with proteinase K at 55 °C overnight. This was followed by chloroform/isoamyl alcohol extraction. Samples of 100µg of isolated DNA were digested to nucleosides with nuclease P1 and alkaline phosphatase (Crain 1990). Analysis of modified DNA base 8-hydroxy-2'-deoxyguanosine (80HdG) was accomplished by UPLC-MS/MS.

### Lipid peroxidation

For isoprostane determination, tissue samples were homogenized in 0.1M phosphate buffer (pH 7.4) + 1mM EDTA + 0.005% butylated hydroxytoluene. Isoprostanes were measured using the 8-isoprostane EIA kit (Cayman Chemical Co.) and following the manufacturer's protocol.

### Protein carbonylation

Carbonylation of proteins, an irreversible oxidative damage, was measured using the Protein Carbonyl Fluorometric Assay Kit (Cayman Chemical Co.) and following the manufacturer's protocol.

### Enzyme activities and metabolite levels

Isolated cells were homogenized in 0.1 M phosphate buffer (pH 7.2) at 4 °C. γ-glu tamylcysteine ligase (GCL), glutathione peroxidase (GPX), catalase (CAT), and superoxide dismutase 1 and 2 (SOD1 and SOD2) activities were measured as previously described in detail (Benlloch 2016). Protein concentration was determined with the Pierce BCA protein assay (Thermo Fisher Scientific). Glutathione (□-L-glutamyl-L-cystenyl-glycine, GSH) was determined by LC/MS as previously reported (Obrador 2014). Cell processing was performed according to published methodology, where rapid N-ethylmaleimide derivatization was used to prevent GSH auto-oxidation (Asensi 1994). NAD⁺ and NADH in isolated cells were quantitated using assay kits from MyBiosource (San Diego, CA).

### RT-PCR and detection of mRNA

Total RNA was isolated using the TRIzol kit from Invitrogen (San Diego, CA) following the manufacturer's instructions. cDNA was obtained using a random hexamer primer and a MultiScribe Reverse Transcriptase kit as recommended by the manufacturer (Taq-Man RT Reagents; Applied Biosystems, Foster City, CA). PCR master mix and AmpliTaq Gold DNA polymerase (Applied Biosystems) were added to the primers previously reported (Benlloch 2016). Real-time quantification of mRNA relative to glyceraldehyde 3-phosphate dehydrogenase (GAPDH) was performed as previously reported (Benlloch 2016).

### Statistics

Data are presented as mean values ± SD for the number of different experiments. Statistical analyses were performed using Student's t-test, and P < 0.05 was considered significant.

### References

Asensi, M. et al. A high-performance liquid chromatography method for measurement of oxidized glutathione in biological samples. Anal Biochem 217, 323-328 (1994).
Berry, M. N., and Friend, D. S. (1969) High-yield preparation of isolated rat liver parenchymal cells: a biochemical and fine structural study. J Cell Biol. 43, 506-520.
Benlloch, M., Obrador, E., Valles, S. L., Rodriguez, M. L., Sirerol, J. A., Alcácer, J., Pellicer, J. A., Salvador, R., Cerdá, C., Sáez, G. T., and Estrela, J. M. (2016) Pterostilbene Decreases the Antioxidant Defenses of Aggressive Cancer Cells In Vivo: A Physiological Glucocorticoids- and Nrf2-Dependent Mechanism. Antioxid. Redox Signal. 24, 974-990.
Boehning, D., Sedaghat, L., Sedlak, T. W. & Snyder, S. H. Heme Oxygenase-2 Is Activated by Calcium-Calmodulin. J. Biol. Chem. 279, 30927-30930 (2004).
Canto C, Sauve AA, Bai P. Crosstalk between poly(ADP-ribose) polymerase and sirtuin enzymes. Mol Aspects Med. 2013 Dec;34(6): 1168-201.
Crain, P. F. Preparation and enzymatic hydrolysis of DNA and RNA for mass spectrometry. Methods Enzymol. 193:782-790; 1990.
Estrela JM, Hernandez R, Terradez P, Asensi M, Puertes IR, and Vina J. Regulation of glutathione metabolism in Ehrlich ascites tumour cells. Biochem J 286 (Pt 1): 257-262, 1992.
Estrela JM, Mena S, Obrador E, Benlloch M, Castellano G, Salvador R, Dellinger RW. Polyphenolic Phytochemicals in Cancer Prevention and Therapy: Bioavailability versus Bioefficacy. J Med Chem. 2017 Dec 14;60(23):9413-9436.
Ferrer P, Asensi M, Segarra R, Ortega A, Benlloch M, Obrador E, Varea MT, Asensio G, Jorda L, and Estrela JM. Association between PTERostilbene and quercetin inhibits metastatic activity of B16 melanoma. Neoplasia 7: 37-47, 2005.
Fischer N, Seo EJ, Efferth T. Prevention from radiation damage by natural products. Phytomedicine. 2018 Aug 1;47:192-200.
Graves CL, Harden SW, LaPato M, Nelson M, Amador B, Sorenson H, Frazier CJ, Wallet SM. A method for high purity intestinal epithelial cell culture from adult human and murine tissues for the investigation of innate immune function. J Immunol Methods. 2014 Dec 1;414:20-31.
Groen, A. K., Sips, H. J., Vervoorn, R. C., and Tager, J. M. (1982) Eur. J. Biochem. 122, 87-93.
Klimberg VS, Souba WW, Dolson DJ, Salloum RM, Hautamaki RD, Plumley DA, Mendenhall WM, Bova FJ, Khan SR, Hackett RL, Bland KI, Copeland III EM. Prophylactic glutamine protects the intestinal mucosa from radiation injury. Cancer. 1990 Jul 1;66(1):62-8.
Kurkjian CJ, Guo H, Montgomery ND, Cheng N, Yuan H, Merrill JR, Sempowski GD, Brickey WJ, Ting JP. The Toll-Like Receptor 2/6 Agonist, FSL-1 Lipopeptide, Therapeutically Mitigates Acute Radiation Syndrome. Sci Rep. 2017 Dec 11;7(1):17355.
Martens CR, Denman BA, Mazzo MR, Armstrong ML, Reisdorph N, McQueen MB, Chonchol M, Seals DR. Chronic nicotinamide riboside supplementation is well-tolerated and elevates NAD+ in healthy middle-aged and older adults. Nat Commun. 2018 Mar 29;9(1):1286.
Mavragani IV, Laskaratou DA, Frey B, Candéias SM, Gaipl US, Lumniczky K, Georgakilas AG. Key mechanisms involved in ionizing radiation-induced systemic effects. A current review. Toxicol Res (Camb). 2015 Aug 11;5(1):12-33.
M'kacher R, El Maalouf E, Terzoudi G, Ricoul M, Heidingsfelder L, Karachristou I, Laplagne E, Hempel WM, Colicchio B, Dieterlen A, Pantelias G, Sabatier L. Detection and automated scoring of dicentric chromosomes in nonstimulated lymphocyte prematurely condensed chromosomes after telomere and centromere staining. Int J Radiat Oncol Biol Phys. 2015 Mar 1;91(3):640-9.
Newsholme, P., Gordon, S., and Newsholme, E. A. (1987) Rates of utilization and fates of glucose, glutamine, pyruvate, fatty acids and ketone bodies by mouse macrophages. Biochem. J. 242, 631-636.
Obrador, E., Valles, S. L., Benlloch, M., Sirerol, J. A., Pellicer, J. A., Alcácer, J., Coronado, J. A.-F., and Estrela, J. M. (2014) Glucocorticoid receptor knockdown decreases the antioxidant protection of B16 melanoma cells: an endocrine systemrelated mechanism that compromises metastatic cell resistance to vascular endothelium-induced tumor cytotoxicity. PloS One. 9, e96466.
Obrador E, Salvador R, Marchio P, López-Blanch R, Jihad-Jebbar A, Rivera P, Vallés SL, Banacloche S, Alcácer J, Colomer N, Coronado JA, Alandes S, Drehmer E, Benlloch M, Estrela JM. Nicotinamide Riboside and Pterostilbene Cooperatively Delay Motor Neuron Failure in ALS SOD1G93A Mice. Mol Neurobiol. 2020 Nov 10. doi: 10.1007/s1 2035-020-02188-7.
Pantelias GE, Maillie HD. A simple method for premature chromosome condensation induction in primary human and rodent cells using polyethylene glycol. Somat Cell Genet 1983;9:533-547.
Patyar RR, Patyar S. Role of drugs in the prevention and amelioration of radiation induced toxic effects. Eur J Pharmacol. 2018 Jan 15;819:207-216.
Riklis E, Kol R, Marko R. Trends and developments in radioprotection: the effect of nicotinamide on DNA repair. Int J Radiat Biol. 1990 Apr;57(4):699-708.
Qi Z, Whitt I, Mehta A, Jin J, Zhao M, Harris RC, et al. Serial determination of glomerular filtration rate in conscious mice using FITC-inulin clearance. Am J Physiol Renal Physiol. 2004;286:F590-596.
Reisz JA, Bansal N, Qian J, Zhao W, Furdui CM. Effects of ionizing radiation on biological molecules-mechanisms of damage and emerging methods of detection. Antioxid Redox Signal 2014, 21: 260-92.
Riley, P.A., 1994. Free radicals in biology: oxidative stress and the effects of ionizing radiation. Int. J. Radiat. Biol. 65, 27-33.
Schmid W. 1975. The micronucleus test. Mutat Res Mutagen Relat Subj. 31: 9-15.
Sirerol JA, Feddi F, Mena S, Rodriguez ML, Sirera P, Aupí M, Perez S, Asensi M, Ortega A, Estrela JM. Topical treatment with pterostilbene, a natural phytoalexin, effectively protects hairless mice against UVB radiation-induced skin damage and carcinogenesis. Free Radic Biol Med. 2015 Aug;85:1-11.
Song Y, Sun H, Xiao J, Wang F, Ding Y, Zhao J, Bai J, Cheng L, Gao K, Liu M, Guo Q, Zhang Y, Gao W, Jia Y, Wen A. Development of a liquid chromatography-tandem mass spectrometric (LC-MS/MS) method for simultaneous determination of epigallocatechin-3-gallate, silibinin, and curcumin in plasma and different tissues after oral dosing of Protandim in rats and its application in pharmacokinetic and tissue distribution studies. J Pharm Biomed Anal. 2019 Jun 5;170:54-62.
Thornton, A. M. (2003) Fractionation of T and B cells using magnetic beads. Curr. Protoc. Immunol. ChaPTER 3, Unit 3.5A.
Wani TA, Shah AG, Wani SM, Wani IA, Masoodi FA, Nissar N, Shagoo MA. Suitability of Different Food Grade Materials for the Encapsulation of Some Functional Foods Well Reported for Their Advantages and Susceptibility. Crit Rev Food Sci Nutr. 2016 Nov 17;56(15):2431-2454
Williams JP, Brown SL, Georges GE, Hauer-Jensen M, Hill RP, Huser AK, Kirsch DG, Macvittie TJ, Mason KA, Medhora MM, Moulder JE, Okunieff P, Otterson MF, Robbins ME, Smathers JB, McBride WH. Animal models for medical countermeasures to radiation exposure. Radiat Res. 2010 Apr;173(4):557-78.
Yoshino J, Imai S-I. Accurate measurement of nicotinamide adenine dinucleotide (NAD+) with high-performance liquid chromatography. Methods Mol Biol. 2013; 1077: 203-215.

### EXAMPLE 2: PT and radioprotective effect

First, the effects of γ radiation and mice survival were studied. Mice received whole-body γ radiation were studied (Figure 1A). Mice received whole-body γ radiation (one single dose; ¹³⁷Cs, 0.5-0.6 Gy/min; Biobeam 8000, GSM GmbH). One Gy= absorption of 1 J of energy/kg of matter. LD50/30 of approx. 7.2 Gy (n=20 per radiation dose).

Next, the thirty-days survival of mice treated with γ rays (LD50/30) and pterostilbene (PT) was analysed (Figure 1B). For that, Swiss albino mice (Charles River, n=20/group) were treated with amifostine (2-(3-aminopropylamino) ethylsulfanylphosphonic acid) (dissolved in physiological saline; one single oral dose 30 min before irradiation; recommended 200 mg/m² in humans was adapted to mice following FDA's guidelines) or pterostilbene (dissolved in 2-hydroxypropyl-b-cyclodextrin, then suspended in 0.3% carboxymethylcellulose; adm. orally, once per day, 5 days before and 2 days after irradiation). Mice received whole-body γ radiation (one single dose of 7.2 Gy of ¹³⁷Cs at 0.5-0.6 Gy/min, equipment= Biobeam 8000, GSM GmbH). One Gy= absorption of 1 J of energy/kg of matter. Data were analyzed with LogRank (Mantel-Cox) test (*comparing all groups vs. controls treated with vehicle). The results in Figure 1B show that the best effect on improving survival (60 days) is exerted by the dose of 100 mg Pterostibene/kg.

Survival of mice treated with γ rays (LD50/30) and resveratrol (Resv) was also analysed and the results are shown in Figure 1C. Swiss albino mice (n= 20/group) were treated with Resvl (dissolved in 2-hydroxypropyl-b-cyclodextrin, then suspended in 0.3% carboxymethylcellulose; adm. orally, once per day, 5 days before and 2 days after irradiation). Mice received whole-body γ radiation (one single dose of 7.2 Gy of ¹³⁷Cs at 0.5-0.6 Gy/min, Mortazavi SMJ et al. Dose-Response 11: 281-292, 2013; equipment= Biobeam 8000, GSM GmbH). One Gy= absorption of 1 J of energy/kg of matter. The results showed that the radioprotective effects of Resv were lower than those of PT.

Plasma and extravascular levels of pterostilbene after its oral administration to mice were studied. Mice were treated with 200 mg Pter/kg b.w. A group of 4-5 animals was sacrificed per each time point. Pter was dissolved in 2-hydroxypropyl-b-cyclodextrin, then suspended in 0.3% carboxymethylcellulose. Nondetectable= n.d. *P< 0.1 comparing data obtained at 10-1440 min versus those found at 5 min. The results are shown in Table 1 below:

| | **Pter (µM)** | | | | |
|---|---|---|---|---|---|
| **Time (min)** | **Plasma** | **Brain** | **Lung** | **Liver** | **Kidney** |
| 5 | 5 ± 2 | 2 ± 1 | 7 ± 2 | 3 ± 1 | 1 ± 0.5 |
| 10 | 18 ± 4* | 6 ± 2* | 15 ± 6 | 8 ± 3* | 4 ± 2* |
| 30 | 55 ± 9* | 10 ± 3* | 23 ± 5* | 11 ± 3* | 6 ± 3* |
| 60 | 98 ± 17* | 24 ± 7* | 35 ± 8* | 17 ± 5* | 10 ± 4* |
| 120 | 46 ± 12* | 14 ± 4* | 21 ± 4* | 12 ± 4* | 8 ± 2* |
| 240 | 21 ± 6* | 8 ± 3* | 12 ± 3* | 7 ± 2* | 2 ± 0.5 |
| 480 | 14 ± 5* | 3 ± 1 | 2 ± 1 | 1 ± 0.5 | n.d. |
| 720 | 1 ± 0.5 | n.d. | n.d. | n.d. | n.d. |
| 1440 | n.d. | n.d. | n.d. | n.d. | n.d. |

These results show that Pterostilbene levels peak, in all tissues studied, at 60 min after administration.

Altogether, the results above confirm the potential of PTER as radioprotector.

### EXAMPLE 3: Combination of PT + SIL

In order to elucidate if pterostilbene could be combined with other polyphenols to achieve a better radioprotective effect, Swiss albino mice (n= 20/group) were treated with pterostilbene and/or other polyphenols. For oral administration PT was solubilized in 2-hydroxypropyl-b-cyclodextrin, then suspended in 0.3% carboxymethylcellulose. All other polyphenolic phytochemicals assayed represent main flavonoids and non-flavonoids, i.e. gallic acid (a phenolic acid), caffeic acid (a hydroxycinnamic acid), curcumin (a curcuminoid), epigallocatechin gallate (a flavanol), genistein (a isoflavone), quercetin (a flavonol), luteolin (a flavone), naringenin (a flavanone), delphindin (an anthocyanidin), and phloridzin (a chalcone), and were solubilized as PT before oral administration. Doses for each phytochemical were selected from the max non-toxic doses published. Data were analyzed with LogRank (Mantel-Cox) test (*comparing all groups vs. controls treated with vehicle, +comparing all groups vs. the group treated with PT alone) (Figure 2).

The results showed that the combination of PT with the different polyphenols resulted in different effects:
a) In some cases, the combination resulted in lower survival than the survival provided the PT administrated separately. This is seen, for instance, in the combination of PT with Gallic acid at 60 days post-irradiation.
b) In some cases, the survival resulted from the combination of the two compounds was similar to that provided by PT administered alone, indicating that the addition of the other compound barely influenced the overall survival. This is seen, for instance, in the combination of PT with Delphinidin at 60 days post-irradiation.
c) In some other cases, the resulting survival equals to approximately the sum of the survivals exerted by the two compounds administered individually. This is seen, for instance, in the combination of PT with Gesnistein at 60 days post-irradiation
d) Lastly, and most importantly, the resulting survival of the combination of the two compounds resulted in a synergistic effect that produced an exceptionally increased survival which cannot be derived from the sum of the effects of the two compounds administered separately. This last case only happened with the combination of Silibinin (SIL) and PT, and happened in both at 30 and 60 days post-irradiation.

Altogether, these results show that the combination of SIL+PT exerts a synergistic radioprotective effect achieving high percentage of survival 30 and 60 days after γ radiation.

In view of this results, the enhanced survival and enhanced radioprotection at longer period of times was studied. Swiss albino mice (n= 20/group) were treated IP with PT (100 mg/kg) and SIL (70 mg/kg) × 7 days (5 days before and 2 days after γ irradiation). For IP administration, a PT salt (pterostilbene phosphate disodium salt, SYNCOM, Groningen, The Netherlands) and SIL salt (silibinin-C-2',3-dihydrogen succinate, disodium salt, Rottapharm/Madaus, Cologne, Germany) were dissolved in sterile vaccination-grade water. Figure 3A shows the Kaplan-Meier curves and LogRank (Mantel-Cox) test (*comparing the PT+SIL-treated group vs. controls treated with vehicle). The IP administration was selected to facilitate the regular administration to a high number of mice. As can be seen in Figure 3A, the combination of PT+SIL provided long term protection, with around 25% (5/20) survival after 1 year post-irradiation time. These results confirm the synergistic enhanced effect of these two compounds when administered in combination and demonstrate that their combined radioprotective effect lasts for long period of times.

Additionally, Figure 3B shows a comparison between administration of PT + SIL before or after the γ irradiation: Swiss albino mice (n= 20/group) were treated IP with PT (100 mg/kg) and SIL (70 mg/kg) × 7 days (5 days before and 2 days after a LD50/30 of γ irradiation, light grey bars; or 7 days after γ irradiation, dark grey bars). Data were analyzed with LogRank (Mantel-Cox) test (*comparing the group treated after irradiation vs. that treated before irradiation). These results show that survival is higher if polyphenols are administered before the radiation.

Next, the effect of pterostilbene (PT) and silibinin (SIL) on the survival of γ irradiated (LD50/30) mice and the effect on body weight: Swiss albino mice (n= 20/group) were treated IP with PT (100 mg/kg) and SIL (70 mg/kg) × 7 days (5 days before and 2 days after γ irradiation). Statistical analyses were performed using Student's t-test (*P< 0.01 comparing PT+SIL vs. vehicle-treated controls). The results are shown in Figure 4A where it can be appreciated that polyphenols reduce the radiation-induced loss in body weight.

The effect of the combined administration of pterostilbene (PT) and silibinin (SIL) on the whole-body radiation-induced alteration of neuromotor functions in mice was also studied: Swiss albino mice (n= 20/group) were treated IP with PT (100 mg/kg) and SIL (70 mg/kg) × 7 days (5 days before and 2 days after γ irradiation). To study the neuromotor function, the standardized Rotarod test was performed as follows: Functions of the test include evaluating balance, grip strength and motor coordination of the subjects; especially in testing the effect of experimental drugs. For this test (Rota Rod, Harvard Apparatus, Holliston, MA) each animal was given three trials and the maximum period (seconds) that it could remain on a rotating axle (3.5 cm diameter; speed of rotation: 15 rpm) without falling was measured. Each mouse was given up to three attempts for an arbitrary limit of 1200 seconds and the longest period was recorded. Changes over time in the different groups were monitored weekly. As shown in Figure 4B, the results showed that neuromotor functions were not significantly altered either by the radiation and/or the administration of PT and SIL.

Next, expression analysis of different antioxidant defense-related enzyme activities in isolated cells were studied by RT-PCR. Statistical analyses were performed using Student's t-test (P< 0.01; *comparing PT+SIL+γ rays-treated mice vs. mice treated with γ rays alone; ⁺comparing PT+SIL-treated mice vs. controls treated with vehicle). These results are shown in Figure 5, and it was concluded that radiation decreases the expression of all the antioxidant defense-related molecules studied, whereas PT and SIL counteract the radiation-induced effects.

Further, the protective effect of PT and SIL on the γ radiation induced oxidative damage was studying by evaluating different molecular markers. To do so, PT, SIL and γ rays were administered as and at the doses previously indicated (n=5 per molecule, cell type, and treatment), and statistical analyses were performed using Student's t-test (P< 0.01; *comparing all groups vs. controls; +comparing comparing PT+SIL+γ rays-treated mice vs. mice treated with γ rays alone). As shown in Figure 6, PT and SIL counteract the radiation-induced increase in different oxidative stress-related biomarkers.

In order to provide a complete analysis of the radioprotective effects of PT and SIL when administered in combination, the antioxidant defence mediated by GCL and GSH was also measured. Thus, PT, SIL and γ rays were administered as and at the doses previously indicated (n=5 per molecule, cell type, and treatment), and statistical analyses were performed using Student's t-test (P< 0.01; *comparing all groups vs. controls; +comparing comparing PT+SIL+γ rays-treated mice vs. mice treated with γ rays alone). As shown in Figure 7, PT and SIL counteract the radiation-induced decrease in different glutathione-related biomarkers.

In Figure 8, catalase and glutathione peroxidase (Fig.8A and B) and superoxide dismutases levels (Fig. 8C and D) were measured when PT, SIL and γ rays were administered as and at the doses previously indicated (n=5 per molecule, cell type, and treatment). Statistical analyses were performed using Student's t-test (P< 0.01; *comparing all groups vs. controls; +comparing comparing PT+SIL+ γ rays-treated mice vs. mice treated with γ rays alone). The results showed that PT and SIL counteract the radiation-induced decrease in different antioxidant enzyme activities.

Lastly, the effect of PT and SIL on γ radiation-induced chromosomal aberrations and micronuclei formation in circulating CD2+ lymphocytes was studied. Telomeres and centromeres of premature chromosome condensation fusions were stained using the Q-FISH technique. The telomeres and centromeres staining was used to score dicentrics, centric rings, and acentric chromosomes from interstitial deletions without telomere staining, acentric chromosomes with only 1 telomere, representing terminal deletions, and acentric chromosomes with 2 telomeres derived from the fusion of 2 acentric chromosomes accompanying the formation of dicentrics or acentric rings. Resulting double-strand DNA breaks (DSBs) were calculated. In the bone marrow double staining was performed using May Grunwald and Giemsa stains. According to this method, polychromatic erythrocytes (PCE) stained reddish-blue and normochromatic erythrocytes (NCE) stained orange, while nuclear materials were dark purple. The micronucleated polychromatic (MnPCE) and normochromatic (MnNCE) cells were counted. Statistical analyses were performed using Student's t-test (P< 0.01; *comparing all groups vs. controls; +comparing PT+SIL+γ rays-treated mice vs. mice treated with γ rays alone). The results are shown in Table 2 below:

| | **Chromosomal Alterations** | | | | | **Micronuclei** | |
|---|---|---|---|---|---|---|---|
| | **No.dicentrics/ 10³ cells** | **No.rings/ 10³ cells** | **No.excess acentrics/ 10³ cells** | **dicentrics+rings/ cell** | **DSB/ 10³ cells** | **MnPCE/ 10³ PCE** | **MnNCE/ 10³ PCE** |
| **Control (no treated)** | **8.9 ± 1.1** | **0 ± 0** | **9.7 ± 1.1** | **0.0089 ± 0.0011** | **0 ± 0** | **4.9 ± 0.7** | **5.3 ± 0.8** |
| **γ-irradiated+vehicle** | **3317 ± 175*** | **597 ± 51*** | **2692 ± 117*** | **3.9 ± 0.2*** | **797 ± 53*** | **209 ± 25*** | **24 ± 4*** |
| **γ-irradiated+PT+SiL** | **1063 ± 130*⁺** | **118 ± 23*⁺** | **608 ± 58*⁺** | **1.2 ± 0.1*⁺** | **133 ± 19*⁺** | **33 ± 9*⁺** | **6 ± 1⁺** |

As can be seen in Table 2, PT and SIL decrease all the radiation-induced cytogenetic alterations, i.e. chromosomal alterations and formation of micronuclei.

### EXAMPLE 4: Combination of PT and SIL with radiomitigators

We next asked whether the combination of radioprotectors (molecules that can reduce the direct damage caused by radiation) and radiomitigators (those that minimize toxicity even after radiation has been delivered) could favour a longer survival. To study this, PT and SIL were combined with the NAD+ booster nicotinamide riboside (NR) and with the toll like receptor 2/6 agonist FSL-1 lipopeptide (FSL-1)

Firstly, the effects of both molecules alone and in combination to confirm their radiomitigation properties were studied. To do so, NR (nicotinamide riboside, 3-aminocarbonyl-1-b-D-ribofuranosyl-pyridinium, Elysium Health, Inc., New York, NY) was dissolved in vaccination-grade water, and the pH of the solution neutralized (pH 7.0) before oral administration (185 mg/kg × day). FSL-1 (fibroblast-stimulating lipopeptide 1, InvivoGen, San Diego, CA) was resuspended in sterile vaccination-grade water, and administered IP (0.25 mg/kg × day, the volume administered was of 50-60 mL depending on the weight of the mouse). NR was administered daily for 30 days, one single dose per day, and starting after the irradiation. FSL-1 was administered only once, 24 h after the irradiation. The total number of mice used was 20. The results are shown in Figure 9, where it is observed that the survival at 60 days post γ irradiation provided by each of these molecules administered alone is 0% for NR, 5% for FSL-1, and 10% for the combination of NR+FSL1.

Next, swiss albino mice (n= 6/group) were treated with PT, SIL, or NR as scheduled previously and at the doses indicated above and statistical analyses were performed using Student's t-test (P< 0.01; *comparing 120 min vs. 30 min; + comparing 2 vs. 0 days; #comparing 3 or 15 days vs. 0 days) and the results are shown in Figure 10. As can be seen in this figure, PT and SIL levels are higher 30 min after their administration, whereas NR-induced NAD+ levels where found similar at the two time points measured (30 and 120 min).

Next, the four compounds PT, SIL, NR and FSL-1 were administered in n=20 mice/group as and at the doses indicated above. Figure 11A shows the administration schedule followed. Figure 11B shows the Kaplan-Meier curves and LogRank (Mantel-Cox) test (*comparing all groups vs. vehicle-treated controls; +comparing the PT+SIL+NR+FSL1-treated group vs. the PT+SIL+NR- or the PT+SIL+FSL1-treated group). Surprisingly, a synergistic effect was also found when NR and FLS1 were combined with PT and SIL, since the survival of the mice was increased for up to a year. Importantly, the combination of the four components provided protection and survival of up to 90% of the mice treated, showing that the combination of the four component achieves long term survival of irradiated mice.

The causes of death in γ irradiated mice (LD50/30) and the effect of the combined administration of the four compounds was studied, as shown in Figure 12. Swiss albino mice (n= 20/group) were treated with PT, SIL, NR and FSL-1 as scheduled previously and at the doses indicated above and the results showed that the full combination (PT+SII+NR+FSL-1) did avoid all deaths related to a) gastrointestinal and hematopoietic ARS, and b) nephropaty. The full combination also minimized deaths related to myeloid leukemia and infections/inflammations.

Next, the γ radiation induced toxicity and the prevention elicited by the combination of PT, SIL, NR and FSL-1 (abbreviated as PSNF) was studied. The results are shown in Table 3 below:

| | | **Control** | **γ-irradiated + vehicle** | **γ-irradiated + PSNF** |
|---|---|---|---|---|
| **Hematology** | | | | |
| | Hematocrit (%) | 39.8 ± 1.3 | 25.9 ± 1.0* | 33.1 ± 2.3*⁺ |
| | Hemoglobin (g/dL) | 12.4 ± 0.4 | 8.1 ± 0.3* | 10.1 ± 0.6*⁺ |
| | Erythrocytes (10⁶/µL) | 8.78 ±0.19 | 6.0 ± 0.3* | 7.68 ± 0.15*⁺ |
| | Platelets (10³/µL) | 452 ± 27 | 135.8 ± 20.7* | 241 ± 21*⁺ |
| | Leukocytes (10³/µL) | 2.7 ± 0.2 | 0.84 ± 0.11* | 1.74 ± 0.11*⁺ |
| | Lymphocytes (10³/µL) | 1.42 ±0.15 | 0.42 ± 0.08* | 0.96 ± 0.11*⁺ |
| | Neutrophiles (10³/µL) | 1.06 ± 0.05 | 0.34 ± 0.05* | 0.56 ± 0.09*⁺ |
| | Monocytes (10³/µL) | 0.09 ± 0.02 | 0.02 ± 0.03* | 0.07 ± 0.03*⁺ |
| | Eosinophiles (10³/µL) | 0.09 ± 0.02 | 0.14 ± 0.05* | 0.09 ± 0.02 |
| | Basophiles (10³/µL) | 0 ± 0 | 0 ± 0 | 0 ± 0 |
| | Bone marrow cell count (10⁶ cells/femur) | 18.8 ± 1.0 | 7.4 ± 0.8* | 10.7 ± 0.9*⁺ |
| | Plasma osmolarity (mOsm/kg) | 270 ± 13 | 240 ± 11* | 265 ± 18⁺ |

| **Clinical chemistry** | | | | |
|---|---|---|---|---|
| | Urea (mg/dL) | 47.4 ± 1.3 | 56.5 ± 1.0* | 47 ± 3⁺ |
| | Uric acid (mg/dL) | 1.78 ±0.08 | 1.28 ± 0.13* | 1.58 ± 0.08⁺ |
| | Total protein (g/dL) | 3.96 ±0.11 | 3.20 ± 0.16* | 3.54 ± 0.05*⁺ |
| | Albumin (g/dL) | 3.06 ±0.11 | 2.74 ± 0.09* | 3.06 ± 0.05 |
| | Creatinine (mg/dL) | 0.40 ± 0.07 | 0.48 ± 0.04 | 0.40 ± 0.07⁺ |
| | Glucose (mg/dL) | 135 ± 3 | 122 ± 4* | 131.4 ± 1.3⁺ |
| | Total bilirubin (mg/dL) | 0.52 ± 0.04 | 0.38 ± 0.04* | 0.48 ± 0.04⁺ |
| | Direct bilirubin (mg/dL) | 0.09 ± 0.02 | 0.06 ± 0.02 | 0.08 ± 0.03 |
| | Aspartate aminotransferase (IU/L) | 145 ± 10 | 264 ± 9* | 197 ± 16*⁺ |
| | Alanine aminotransferase (IU/L) | 7.1 ± 0.3 | 50 ± 4* | 28 ± 4*⁺ |
| | γ-glutamyl-transpeptidase (IU/L | 1.82 ± 0.13 | 3.5 ± 0.3* | 2.12 ± 0.19⁺ |
| | Alkaline phosphatase (IU/L) | 122 ± 7 | 163 ± 8* | 136 ± 12⁺ |
| | Lactate dehydrogenase (IU/L) | 197 ± 10 | 363 ± 14* | 246 ± 10*⁺ |
| | Sodium (mEq/L) | 143 ± 6 | 119 ± 5* | 138 ± 5⁺ |
| | Potassium (mEq/L) | 8.04 ± 0.11 | 10.6 ± 0.4* | 8.7 ± 0.4⁺ |
| | Chloride (mEq/L) | 95 ± 4 | 114 ± 5* | 100 ± 2⁺ |

| **Isolated CD2⁺ lymphocytes** | | | | |
|---|---|---|---|---|
| | GSH (nmol/10⁶ cells) | 5.5 ± 0.2 | 3.02 ± 0.19* | 4.40 ± 0.16*⁺ |
| | Cell volume (µm³) | 176 ± 5 | 196 ± 6* | 182 ± 4⁺ |
| | Glucose utilization (µmol/g × min) | 1.10 ± 0.07 | 0.60 ± 0.10* | 1.00 ± 0.07⁺ |
| | Glutamine utilization (µmol/g × min) | 3.32 ± 0.08 | 2.02 ± 0.13* | 2.98 ± 0.08*⁺ |

As can be seen in Table 3, treatment with the full combination (PT+SII+NR+FSL-1) helps to improve the radiation-induced toxicity.

Lastly, to elucidate if the treatment with the four combined compounds (PT + SIL + NR + FSL-1; PSNF) would interfere with the therapeutic efficacy of anti-cancer radiotherapy (X rays) in tumours xenografts, female nu/nu nude mice (ages 6-8 weeks) (n= 5 mice/group) were inoculated s.c. with 5 x10⁶ A549 (lung adenocarcinoma) or MDA-MB-231 (triple negative mammary carcinoma) cells per mouse. Tumor volume was calculated based on two dimensions, measured using calipers, and was expressed in cubic millimeters according to the formula: V = 0.5a × b², where a and b are the long and short diameters of the tumor, respectively. Tumors were irradiated with X-rays (10 Gy) using a 6-keV SL75 linear accelerator from Philips. Single fraction radiotherapy was administered at a rate of 5.0 Gy/min. The protocol for PSNF administration was identical to that explained above, and the radiotherapy was administered on day 21. Statistical analyses were performed using Student's t-test (P< 0.01; *comparing all groups vs. controls; +comparing the γ rays+PSNF-treated group vs. the group treated with γ rays alone).

The results are shown in Figure 13, where can be seen that the combination of the four compounds does not diminish the efficacy of anti-cancer treatment with X rays, proving the feasibility of using these compounds in patients undergoing said radiotherapy treatment.

Altogether, these results support the use of compositions comprising at least PT and SIL, and optionally NR and FSL1, to protect and mitigate from the damage induced by radiation. It is important to note that the radioprotective of these compounds was extended for long period of times (1 year or longer) as shown in Figure 3A and Figure 11B.

## Claims

1. Pterostilbene, or a pharmaceutically acceptable salt thereof, **for use** in the prevention, amelioration or reduction of diseases induced by ionizing radiation in a subject, in combination with Silibinin, or a pharmaceutically acceptable salt thereof, wherein the administration is carried out before the radiation, or after the radiation, or during the radiation; and wherein the combination is herein understood as the simultaneous or sequential administration, in any order, of a) Pterostilbene, or a pharmaceutically acceptable salt thereof, and b) Silibinin, or a pharmaceutically acceptable salt thereof, to said subject.

2. Pterostilbene for use according to claim 1, wherein Pterostilbene and Silibinin, or pharmaceutically acceptable salts thereof, are administered in a sole pharmaceutical composition or in separate pharmaceutical compositions.

3. Pterostilbene for use according to any of claims 1 to 2, wherein the administration of Pterostilbene and Silibinin, or pharmaceutically acceptable salts thereof, is topical, oral or parenteral.

4. Pterostilbene for use according to any of claims 1 to 3, wherein Pterostilbene and Silibinin, or pharmaceutically acceptable salts thereof, are administrated several times simultaneously or sequentially before and/or after radiation, or both.

5. Pterostilbene for use according to any of claims 1 to 4, wherein the combination is in a dosage capable of providing a radioprotective effect.

6. Pterostilbene for use according to any of claims 1 to 5, wherein Pterostilbene, or a pharmaceutically acceptable salt thereof, is administered at a dosage from 300 mg/kg to 30 mg/kg, preferably 100 mg/kg, and Silibinin, or a pharmaceutically acceptable salt thereof, is administered at a dosage from 200mg/kg to 35 mg/kg of Silibinin, preferably 70 mg/kg.

7. Pterostilbene for use according to any of claims 1 to 6, wherein the use further comprises the administration in combination with at least one or more radiomitigator compound/s, wherein the combination is herein understood as the simultaneous or sequential administration of a) Pterostilbene, or a pharmaceutically acceptable salt thereof, b) Silibinin, or a pharmaceutically acceptable salt thereof, and c) at least one or more radiomitigator compound/s, or any pharmaceutically acceptable salt thereof, to said subject.

8. Pterostilbene for use according to claim 7, wherein the at least one or more radiomitigator compound/s is a NAD+ booster and/or a Fibroblast-Stimulating Lipopeptide, or any pharmaceutically acceptable salts thereof.

9. Pterostilbene for use according to claim 7, wherein the use further comprises the administration in combination with at least two or more radiomitigator compounds, wherein the at least two or more radiomitigator compounds are NAD+ booster and Fibroblast-Stimulating Lipopeptide, or any pharmaceutically acceptable salts thereof.

10. Pterostilbene for use according to any of claims 7 to 9, wherein the NAD+ booster, or any pharmaceutically acceptable salt thereof, is administered at a dosage from 400 mg/kg to 100 mg/kg, preferably 185 mg/kg, and/or wherein the Fibroblast-Stimulating Lipopeptide, or a pharmaceutically acceptable salt thereof, is administered at a dosage from 1mg/kg to 0,1 mg/kg, preferably 0.25 mg/kg.

11. Pterostilbene for use according to any of the claims 8 to 10, wherein the NAD+ booster is Nicotinamide Riboside.

12. Pterostilbene for use according to any of claims 1 to 11, wherein the diseases induced by ionizing radiation are related to DNA damage, radiation-induced cytotoxicity, genotoxicity and/or oxidative cellular damage.

13. Pterostilbene for use according to any of claims 1 to 12, wherein the diseases induced by ionizing radiation are acute radiation syndrome and/or chronic radiation syndrome.

14. Pterostilbene for use according to any of claims 1 to 13, wherein the ionizing radiation is gamma rays or X-rays.

15. Pterostilbene for use according to any of claims 1-14, wherein the Pterostilbene is Pterostilbene phosphate disodium salt and/or wherein the Silibinin is silibinin-C-2',3-dihydrogen succinate, disodium salt.

16. A **pharmaceutical composition** comprising a) Pterostilbene, or a pharmaceutically acceptable salt thereof, and b) Silibinin, or a pharmaceutically acceptable salt thereof.

17. The pharmaceutical composition according to claim 16, wherein the dosage of Pterostilbene, or a pharmaceutically acceptable salt thereof, present in said composition is from 300 mg/kg to 30 mg/kg, preferably 100 mg/kg, and wherein the dosage of Silibinin, or a pharmaceutically acceptable salt thereof, present in said composition is from 200mg/kg to 35 mg/kg of Silibinin, preferably 70 mg/kg.

18. The pharmaceutical composition according to any of claims 16 and 17, wherein the pharmaceutical composition further comprises at least one or more radiomitigator compound/s, preferably selected from
a. NAD+ booster, and/or
b. Fibroblast-Stimulating Lipopeptide-1,
or any pharmaceutically acceptable salts thereof.

19. The pharmaceutical composition according to claim 18, wherein the pharmaceutical composition further comprises at least two or more radiomitigators compounds, wherein the at least two or more radiomitigator compounds are a NAD+ booster and Fibroblast-Stimulating Lipopeptide-1, or any pharmaceutically acceptable salts thereof.

20. The pharmaceutical composition according to any of claims 18 to 19, wherein the NAD+ booster, or a pharmaceutically acceptable salt thereof, is present in said composition at a dosage from 400 mg/kg to 100 mg/kg, preferably 185 mg/kg, and/or wherein the Fibroblast-Stimulating Lipopeptide-1, or a pharmaceutically acceptable salt thereof, is present in said composition at a dosage from 1mg/kg to 0,1 mg/kg, preferably 0.25 mg/kg.

21. The pharmaceutical composition according to any of claims 18 to 20, wherein the NAD+ booster is Nicotinamide Riboside.

22. The pharmaceutical composition according to any of claims 16 to 21, wherein said composition further comprises a pharmaceutically acceptable carrier, additive and/or excipient.

23. The pharmaceutical composition according to any of claims 16-22, wherein the Pterostilbene is Pterostilbene phosphate disodium salt and/or wherein the Silibinin is silibinin-C-2',3-dihydrogen succinate, disodium salt.

24. A **kit of parts** comprising at least two recipients comprising at least Pterostilbene and Silibinin, or any pharmaceutically acceptable salts thereof, and optionally at least one or more radiomitigator compound/s.

25. The kit of parts according to claim 24, wherein the Pterostilbene, or a pharmaceutically acceptable salt thereof, is present at a dosage from 300 mg/kg to 30 mg/kg, preferably 100 mg/kg, and Silibinin, or a pharmaceutically acceptable salt thereof, is present at a dosage from 200mg/kg to 35 mg/kg of Silibinin, preferably 70 mg/kg.

26. The kit of parts according to claims 24 and 25, wherein the optionally at least one or more radiomitigator compound/s is selected from the group of:
a. NAD+ boosters, and/or
b. A Fibroblast-Stimulating Lipopeptide,
or any pharmaceutically acceptable salts thereof.

27. The kit of parts according to claim 26, wherein the NAD+ boosters, or a pharmaceutically acceptable salt thereof, is present at a dosage from 400 mg/kg to 100 mg/kg, preferably 185 mg/kg, and/or wherein the Fibroblast-Stimulating Lipopeptide, or a pharmaceutically acceptable salt thereof, is present at a dosage from 1mg/kg to 0,1 mg/kg, preferably 0.25 mg/kg.

28. The kit of parts according to any of the claims 26 and 27, wherein the NAD+ booster is Nicotinamide Riboside.

29. The kit of parts according to any of the claims 24-27, wherein the Pterostilbene is Pterostilbene phosphate disodium salt and/or wherein the Silibinin is silibinin-C-2',3-dihydrogen succinate, disodium salt.

30. A **method** for preventing, ameliorating or reducing diseases induced by ionizing radiation in a subject, comprising administering Pterostilbene in combination with Silibinin, or pharmaceutically acceptable salts thereof, wherein said administration is performed before the radiation, or after the radiation, or during the radiation; and wherein the combination is herein understood as the simultaneous or sequential administration of a) Pterostilbene, or a pharmaceutically acceptable salt thereof, and b) Silibinin, or a pharmaceutically acceptable salt thereof, to said subject.

31. The method of claim 30, further comprising the administration of at least one radiomitigator compound, wherein the at least one or more radiomitigator compound are preferably selected from
a. NAD+ boosters, and/or
b. Fibroblast-Stimulating Lipopeptide,
or any pharmaceutically acceptable salts thereof.

32. The method according to claim 31, wherein the NAD+ booster is Nicotinamide Riboside.
